# EUROPEAN PATENT SPECIFICATION

(11) **EP 0 724 447 B1**
(45) Date of publication and mention of the grant of the patent: **07.05.2003**
(21) Application number: 92923139.7
(22) Date of filing: 23.10.1992
(51) Int. Cl.: A61K 31/70, C07H 21/00

(54) **DERIVATIZED OLIGONUCLEOTIDES HAVING IMPROVED UPTAKE**
DERIVATISIERTE OLIGONUKLEOTIDE MIT VERBESSERTEM AUFNAHMEVERMÖGEN
OLIGONUCLEOTIDES DERIVES PRESENTANT UNE MEILLEURE FACILITE D'ABSORPTION

(30) Priority: 24.10.1991 US 782374
(43) Date of publication of application: 07.08.1996
(73) Proprietor: ISIS PHARMACEUTICALS, INC., Carlsbad, CA 92008 (US)
(72) Inventor: MANOHARAN, Muthiah, Carlsbad, CA 92008 (US); COOK, Philip, Dan, Carlsbad, CA 92009 (US); BENNETT, Clarence Frank, Carlsbad, CA 92008 (US)
(74) Representative: Hallybone, Huw George
(86) International application number: US9209196
(87) International publication number: WO93007883

(56) References cited:
- WO-A-91/10671
- WO-A-91/14696
- WO-A-94/06815
- WO-A-95/06659
- US-A- 4 958 013
- BIOCONJUGATE CHEMISTRY, vol. 1, no. 3, 1 May 1990 pages 165-187, XP 000174627 GOODCHILD J 'CONJUGATES OF OLIGONUCLEOTIDES AND MODIFIED OLIGONUCLEOTIDES: A REVIEW OF THEIR SYNTHESIS AND PROPERTIES'
- BIOCONJUGATE CHEMISTRY, vol. 2, no. 6, 1 November 1991 pages 464-465, XP 000235497 CHING-HSUAN TUNG ET AL 'PREPARATION OF OLIGONUCLEOTIDE-PEPTIDE CONJUGATES'
- ANGEWANDTE CHEMIE. INTERNATIONAL EDITION, vol. 30, no. 6, 1 June 1991 pages 613-629, XP 000218347 ENGLISCH U ET AL 'CHEMICALLY MODIFIED OLIGONUCLEOTIDES AS PROBES AND INHIBITORS'
- TETRAHEDRON LETT. (1991), 32(49), 7171-4 CODEN: TELEAY;ISSN: 0040-4039, 1991 MANOHARAN, MUTHIAH ET AL 'Novel functionalization of the sugar moiety of nucleic acids for multiple labeling in the minor groove'
- TETRAHEDRON LETTERS, vol. 32, no. 51, 16 December 1991 pages 7511-7512, XP 000247164 LESZEK CZUCHAJOWSKI 'PORPHYRINYL-URIDINES AS THE FIRST WATER SOLUBLE PORPHYRINYL- NUCLEOSIDES'
- DATABASE EMBASE ELSEVIER SCIENCE PUBLISHERS, AMSTERDAM, NL AN=93054753, & ANN. NEW YORK ACAD. SCI., vol. 660, 1992 pages 306-309, MANOHARAN M. ET AL. 'CHEMICAL MODIFICATIONS TO IMPROVE UPTAKE AND BIOAVAILABILTY OF ANTISENSE OLIGONUCLEOTIDES.'
- JOURNAL OF THE AMERICAN CHEMICAL SOCIETY, Volume 104, issued 06 October 1982, RAMIREZ et al., "Nucleotidophospholipids: Oligonucleotide Derivatives with Membrane-Recognition Groups", pages 5483-5486.
- JOURNAL OF THE AMERICAN SOCIETY, Volume 111, issued 01 July 1989, TELSER et al., "Synthesis and Characterization of DNA Oligomers and Duplexes Containing Covalently Attached Molecular Lables: Comparison of Biotin, Flurescein, and Pyrene Labels by Thermodynamic and Optical Spectroscopic Measures", pages 6966-6976.

## Description

### FIELD OF THE INVENTION

This application is directed to sequence specific oligonucleotides that include functionalized nucleosides having substituents such as steroids, reporter enzymes, peptides, or proteins attached via linking groups.

### BACKGROUND OF THE INVENTION

Messenger RNA (mRNA) directs protein synthesis. Antisense methodology is the complementary hybridization of relatively short oligonucleotides to mRNA or DNA such that the normal, essential functions of these intracellular nucleic acids are disrupted. Hybridization is the sequence-specific hydrogen bonding of oligonucleotides to RNA or single-stranded DNA via complementary Watson-Crick base pairs.

The naturally occurring events that provide the disruption of the nucleic acid function, discussed by Cohen in *Oligonucleotides: Antisense Inhibitors of Gene Expression,* CRC Press, Inc., Boca Raton, F1 (1989), are thought to be of two types. The first, hybridization arrest, denotes a terminating event in which the oligonucleotide inhibitor binds to the target nucleic acid and thus prevents, by simple steric hindrance, the binding of essential proteins, most often ribosomes, to the nucleic acid. Methyl phosphonate oligonucleotides (*see, e.g*., Miller, *et al., Anti-Cancer Drug Design* **1987,** *2*, 117) and α-anomer oligonucleotides, the two most extensively studied antisense agents, are thought to disrupt nucleic acid function by hybridization arrest.

The second type of terminating event for antisense oligonucleotides involves the enzymatic cleavage of targeted RNA by intracellular RNase H. A 2'-deoxyribofuranosyl oligonucleotide or oligonucleotide analog hybridizes with the targeted RNA to form a duplex that activates the RNase H enzyme to cleave the RNA strand, thus destroying the normal function of the RNA. Phosphorothioate oligonucleotides provide the most prominent example of antisense agents that operate by this type of antisense terminating event.

Considerable research is being directed to the application of oligonucleotides and oligonucleotide analogs as antisense agents for diagnostics, research reagents, and therapeutics. At least for therapeutic purposes, the antisense oligonucleotides and oligonucleotide analogs must be transported across cell membranes or taken up by cells to express activity. One method for increasing membrane or cellular transport is by the attachment of a pendant lipophilic group.

Ramirez, *et al., J. Am. Chem. Soc.* **1982,** *104*:, 5483, introduced the phospholipid group 5'-O-(1,2-di-O-myristoyl-sn-glycero-3-phosphoryl) into the dimer TpT independently at the 3' and 5' positions. Subsequently Shea, *et al*., *Nuc. Acids Res.* **1990,** *18*, 3777, disclosed oligonucleotides having a 1,2-di-O-hexyldecyl-rac-glycerol group linked to a 5'-phosphate on the 5'-terminus of the oligonucleotide. Certain of the Shea, *et*. *al.* authors disclosed these and other compounds in patent application PCT/US90/01002. Another glucosyl phospholipid was disclosed by Guerra, *et al., Tetrahedron Letters* **1987,** *28*, 3581.

In other work, a cholesteryl group was attached to the inter-nucleotide linkage between the first and second nucleotides (from the 3' terminus) of an oligonucleotide. This work is disclosed in United States Patent No. 4,958,013 and by Letsinger, *et al., Proc. Natl. Acad. Sci. USA* **1989,** *86*, 6553. The aromatic intercalating agent anthraquinone was attached to the 2' position of a sugar fragment of an oligonucleotide as reported by Yamana, *et al., Bioconjugate Chem.* **1990,** *1*, 319.

Lemairte, *et al., Proc. Natl. Acad. Sci. USA* **1986,** *84*, 648 and Leonetti, *et al., Bioconjugate Chem.* 1990, *1*, 149, disclose modifying the 3' terminus of an oligonucleotide to include a 3'-terminal ribose sugar moiety. Poly(L-lysine) was linked to the oligonucleotide via periodate oxidation of this terminal ribose followed by reduction and coupling through a N-morpholine ring. Oligonucleotide-poly(L-lysine) conjugates are described in European Patent application 87109348.0, wherein the lysine residue was coupled to a 5' or 3' phosphate of the 5' or 3' terminal nucleotide of the oligonucleotide. A disulfide linkage has also been utilized at the 3' terminus of an oligonucleotide to link a peptide to the oligonucleotide, as described by Corey, *et al., Science* **1987,** *238*, 1401; Zuckermann, *et al., J. Am. Chem. Soc.* **1988,** *110*, 1614; and Corey, *et al., J. Am. Chem. Soc.* **1989,** *111*, 8524.

Nelson, et *al., Nuc. Acids Res.* **1989,** *17,* 7187 describe a linking reagent for attaching biotin to the 3'-terminus of an oligonucleotide. This reagent, N-Fmoc-O-DMT-3-amino-1,2-propanediol, is commercially available from Clontech Laboratories (Palo Alto, CA) under the name 3'-Amine on and from Glen Research Corporation (Sterling, VA) under the name 3'-Amino-Modifier. This reagent was also utilized to link a peptide to an oligonucleotide, as reported by Judy, *et al., Tetrahedron Letters* **1992,** *32*, 879. A similar commercial reagent (actually a series of linkers having various lengths of polymethylene connectors) for linking to the 5'-terminus of an oligonucleotide is 5'-Amino-Modifier C6, also from Glen Research Corporation. These compounds or similar ones were utilized by Krieg, *et al., Antisense Research and Development* **1991,** *1*, 161 to link fluorescein to the 5'-terminus of an oligonucleotide. Other compounds of interest have also been linked to the 3'-terminus of an oligonucleotide. Asseline, *et al., Proc. Natl. Acad. Sci. USA* **1984,** *81*, 3297 described linking acridine on the 3'-terminal phosphate group of an poly (Tp) oligonucleotide via a polymethylene linkage. Haralambidis, *et al., Tetrahedron Letters* **1987,** *28*, 5199 reported building a peptide on a solid state support and then linking an oligonucleotide to that peptide via the 3' hydroxyl group of the 3' terminal nucleotide of the oligonucleotide. Chollet, *Nucleosides & Nucleotides* **1990,** *9*, 957 attached an Aminolink 2 (Applied Biosystems, Foster City, CA) to the 5' terminal phosphate of an oligonucleotide. They then used the bifunctional linking group SMPB (Pierce Chemical Co., Rockford, Il) to link an interleukin protein to the oligonucleotide.

An EDTA iron complex has been linked to the 5 position of a pyrimidine nucleoside as reported by Dreyer, *et al., Proc. Natl. Acad. Sci. USA* **1985,** *82*, 968. Fluorescein has been linked to an oligonucleotide in the same manner, as reported by Haralambidis, *et al., Nucleic Acid Research* **1987,** *15*, 4857 and biotin in the same manner as described in PCT application PCT/US/02198. Fluorescein, biotin and pyrene were also linked in the same manner as reported by Telser, *et al., J. Am. Chem. Soc.* **1989,** *111*, 6966. A commercial reagent, Amino-Modifier-dT from Glen Research Corporation, can be utilized to introduce pyrimidine nucleotides bearing similar linking groups into oligonucleotides.

Cholic acid linked to EDTA for use in radioscintigraphic imaging studies was reported by Betebenner, *et al., Bioconjugate Chem.* **1991,** *2*, 117; however, it is not known to link cholic acid to nucleosides, nucleotides or oligonucleotides.

WO 91/14696 discloses a composition for enhancing the delivery of an oligonucleotide into a cell comprising an oligonucleotide bound to a transport agent via a molecular linker. The composition can be used for therapy and diagnosis.

*Angewandte Chemie,* International Edition, Volume 30 (No. 6), June **1991,** pages 613-629 discloses various oligonucleotides comprising functionalized nucleosides, and their use in diagnostic and antisense therapy.

*Tetrahedron Letters,* Volume 32 (No. 49), **1991,** pages 7171-7174, discloses oligonucleotides comprising modified adenosine, the sugar ring of which is alkylated in 2'-O position in order to allow the addition of a reporter group or a therapeutic agent at the 2'-position.

A review article in *Bioconjugate Chem.* (Volume 1 (No.3), May **1990,** pages 165-187) generally describes certain chemical groups used in conjugates of oligonucleotides.

The aforementioned references do not, however, describe a compound comprising a plurality of linked nucleosides as claimed in the present application.

It is an object of this invention to provide sequence-specific oligonucleotides having improved transfer across cellular membranes.

It is a further object of this invention to provide improvements in research and diagnostic methods and materials for assaying bodily states in animals, especially disease states.

It is an additional object of this invention to provide therapeutic and research materials having improved transfer and uptake properties for the treatment of diseases through modulation of the activity of RNA or DNA.

In accordance with these and other objects evident from the specification, there is provided a compound comprising a plurality of linked nucleosides, wherein:
at least one of said nucleosides is functionalized at its 2'-position by direct attachment of a substituent selected from the group consisting of a steroid molecule, a reporter enzyme, a peptide, a protein, a pyrene, a water soluble vitamin, a lipid soluble vitamin, a metal chelator, a porphyrin, an alkylator, a hybrid photonuclease/intercalator, and an aryl azide photo-crosslinking agent, or
at least one of said nucleosides is functionalized at its 2'-position by attachment, through a linking moiety, of a substituent selected from the group consisting of a reporter enzyme, a peptide, a protein, a water soluble vitamin, a lipid soluble vitamin, an RNA cleaving complex, a metal chelator, an alkylator, and a hybrid photonuclease/intercalator, provided that said linking moiety does not include a disulfide group.
In certain preferred embodiments of the invention, the substituents comprise a steroid molecule, biotin, a reporter enzyme or a fluorescein dye molecule. In these embodiments, the steroid molecule is selected from the group consisting of cholic acid, deoxycholic acid, dehydrocholic acid, cortisone, testosterone, cholesterol and digoxigenin with the most preferred steroid molecule being cholic acid. Preferred reporter enzymes include horseradish peroxidase and alkaline phosphatase.

In other preferred embodiments, peptides or proteins attached to the 2'-position comprise sequence-specific peptides and sequence-specific proteins, including phosphatases, peroxidases and nucleases.

The linking molecules of the invention may comprise Ω-aminoalkoxy linkers, Ω-aminoalkylamino linkers, heterobifunctional linkers or homobifunctional linkers. A particularly preferred linking molecule of the invention is a 5-aminopentoxy group.

In preferred embodiments of the invention at least a portion of the linked nucleosides are 2'-deoxy-2'-fluoro, 2'-methoxy, 2'-ethoxy, 2'-propoxy, 2'-aminoalkoxy or 2'-allyloxy nucleosides. In other preferred embodiments of the invention the linked nucleosides are linked with phosphorothioate linking groups.

The invention also provides compounds that have a plurality of linked nucleosides. In preferred embodiments, at least one of the nucleosides is: (1) a 2'-functionalized nucleoside having cholic acid linked to its 2'-position; (2) a heterocyclic base functionalized nucleoside having cholic acid linked to its heterocyclic base; (3) a 5' terminal nucleoside having cholic acid linked to its 5'-position; (4) a 3' terminal nucleoside having cholic acid linked to its 3'-position; or (5) an inter-strand nucleoside having cholic acid linked to an inter-stand linkage linking said inter-strand nucleoside to an adjacent nucleoside.

In certain embodiments of the invention having linked nucleosides, at least one linked nucleosides bears a 2'-deoxy'-2'-fluoro, 2'-O-C₁-C₂₀-alkyl, 2'-O-C₂-C₂₀-alkenyl, 2'-O-C₂-C₂₀-alkynyl, 2'-S-C₁-C₂₀-alkyl, 2'-S-C₂-C₂₀-alkenyl, 2'-S-C₂-C₂₀-alkynyl, 2'-NH-C₁-C₂₀-alkyl, 2'-NH-C₂-C₂₀-alkenyl, or 2'-NH-C₂-C₂₀-alkynyl substituent.

The invention also provides a method for enhancing the binding affinity and/or stability of an antisense oligonucleotide comprising functionalizing the oligonucleotide in accordance with the method described is Claim 9 hereinafter

### DETAILED DESCRIPTION OF THE INVENTION

Antisense therapeutics can be practiced in a variety of organisms ranging from unicellular prokaryotic and eukaryotic organisms to multicellular eukaryotic organisms. Any organism that utilizes DNA-RNA transcription or RNA-protein translation as a fundamental part of its hereditary, metabolic or cellular control is susceptible to antisense therapeutics and/or prophylactics. Seemingly diverse organisms such as bacteria, yeast, protozoa, algae, all plant and all higher animal forms, including warm-blooded animals, can be treated by antisense therapy. Further, since each of the cells of multicellular eukaryotes also includes both DNA-RNA transcription and RNA-protein translation as an integral part of its cellular activity, antisense therapeutics and/or diagnostics can also be practiced on such cellular populations. Furthermore, many of the organelles, *e.g.*, mitochondria and chloroplasts, of eukaryotic cells also include transcription and translation mechanisms. As such, single cells, cellular populations or organelles can also be included within the definition of organisms that are capable of being treated with antisense therapeutics or diagnostics. As used herein, therapeutics is meant to include both the eradication of a disease state, killing of an organism, *e.g*., bacterial, protozoan or other infection, or control of erratic or harmful cellular growth or expression.

While we do not wish to be bound by any particular theory, it is believed that the presence of many nuclear proteins in the nucleus is due to their selective entry through the nuclear envelope rather than to their selective retention within the nucleus after entry. By this mechanism, the nucleus is able to selectively take up certain proteins and not others. The uptake is based upon the sequence of the peptide or protein, which provides a selective signal sequence that allows accumulation of the peptide or protein in the nucleus. One such peptide signal sequence is found as part of the SV40 large T-antigen. *See, e.g.,* Dingwell, *et al. Ann. Rev. Cell Bio.* **1986,** *2*, 367; Yoneda, *et al., Experimental Cell* *Research* **1987,** *170*, 439; and Wychowski, *et al., J. Virol.* **1986,** *61*, 3862.

According to the present invention a substituent such as a steroid molecule, a reporter molecule, a non-aromatic lipophilic molecule, a reporter enzyme, a peptide, a protein, a water soluble vitamin, a lipid soluble vitamin, an RNA cleaving complex, a metal chelator, a porphyrin, an alkylator, a hybrid photonuclease/intercalator, or an aryl azide photo-crosslinking agent is attached to at least one nucleoside in an antisense diagnostic or therapeutic agent to assist in the transfer of the antisense therapeutic or diagnostic agent across cellular membranes. Such antisense diagnostic or therapeutic agent is formed from a plurality of linked nucleosides of a sequence that is "antisense" to a region of an RNA or DNA that is of interest. Thus, one or more nucleoside of the linked nucleosides are "functionalized" to include a substituent linked to the nucleoside via a linking group. For the purposes of identification, such functionalized nucleosides can be characterized as substituent-bearing (*e.g.*, steroid-bearing) nucleosides. Linked nucleosides having at least one functionalized nucleoside within their sequence demonstrate enhanced antisense activity when compared to linked nucleoside that do not contain functionalized nucleoside. These "functionalized" linked nucleosides further demonstrate increased transfer across cellular membranes.

For the purposes of this invention. the terms "reporter molecule" and "reporter enzyme" include molecules or enzymes having physical or chemical properties that allow them to be identified in gels, fluids, whole cellular systems, broken cellular systems, and the like utilizing physical properties such as spectroscopy, radioactivity, colorimetric assays, fluorescence, and specific binding. Steroids include chemical compounds that contain a perhydro-1,2-cyclopentanophenanthrene ring system. Proteins and peptides are utilized in their usual sense as polymers of amino acids. Normally peptides are amino acid polymers that contain a fewer amino acid monomers per unit molecule than proteins. Non-aromatic lipophilic molecules include fatty acids, esters, alcohols and other lipid molecules, as well as synthetic cage structures such as adamantane and buckminsterfullerenes that do not include aromatic rings within their structure.

Particularly useful as steroid molecules are the bile acids, including cholic acid, deoxycholic acid and dehydrocholic acid. Other useful steroids are cortisone, digoxigenin, testosterone, cholesterol and cationic steroids such as cortisone having a trimethylaminomethyl hydrazide group attached via a double bond at the 3 position of the cortisone rings. Particularly useful reporter molecules are biotin and fluorescein dyes. Particularly useful non-aromatic lipophilic molecules are alicyclic hydrocarbons, saturated and unsaturated fatty acids, waxes, terpenes, and polyalicyclic hydrocarbons, including adamantane and buckminsterfullerenes. Particularly useful reporter enzymes are alkaline phosphatase and horseradish peroxidase. Particularly useful peptides and proteins are sequence-specific peptides and proteins, including phosphodiesterase, peroxidase, phosphatase, and nuclease proteins. Such peptides and proteins include SV40 peptide, RNase A, RNase H and Staphylococcal nuclease. Particularly useful terpenoids are vitamin A, retinoic acid, retinal, and dehydroretinol.

Vitamins according to the invention generally can be classified as water soluble or lipid soluble. Water soluble vitamins include thiamine, riboflavin, nicotinic acid or niacin, the vitamin B₆ pyridoxal group, pantothenic acid, biotin, folic acid, the B₁₂ cobamide coenzymes, inositol, choline and ascorbic acid. Lipid soluble vitamins include the vitamin A family, vitamin D, the vitamin E tocopherol family and vitamin K (and phytols). The vitamin A family, including retinoic acid and retinol, are absorbed and transported to target tissues through their interaction with specific proteins such as cytosol retinol-binding protein type II (CRBP-II), Retinol-binding protein (RBP), and cellular retinol-binding protein (CRBP). These proteins, which have been found in various parts of the human body, have molecular weights of approximately 15 kD. They have specific interactions with compounds of vitamin-A family, especially, retinoic acid and retinol.

The vitamin A family of compounds can be attached to oligonucleotides via acid or alcohol functionalities found in the various family members. For example, conjugation of an N-hydroxy succinimide ester of an acid moiety of retinoic acid to an amine function on a linker pendant to an oligonucleotide resulted in linkage of vitamin A compound to the oligonucleotide via an amide bond. Also, retinol was converted to its phosphoramidite, which is useful for 5' conjugation.

α-Tocopherol (vitamin E) and the other tocopherols (beta through zeta) can be conjugated to oligonucleotides to enhance uptake because of their lipophilic character. Also, the lipophilic vitamin, vitamin D, and its ergosterol precursors can be conjugated to oligonucleotides through their hydroxyl groups by first activating the hydroxyls groups to, for example, hemisuccinate esters. Conjugation then is effected to an aminolinker pendant from the oligonucleotide. Other vitamins that can be conjugated to oligonucleotide aminolinkers through hydroxyl groups on the vitamins include thiamine, riboflavin, pyridoxine, pyridoxamine, pyridoxal, deoxypyridoxine. Lipid soluble vitamin K's and related quinone-containing compounds can be conjugated via carbonyl groups on the quinone ring. The phytol moiety of vitamin K may also serve to enhance bind of the oligonucleotides to cells.

Pyridoxal (vitamin B₆) has specific B₆-binding proteins. The role of these proteins in pyridoxal transport has been studied by Zhang and McCormick, *Proc. Natl. Acad. Sci. USA*, **1991** *88*, 10407. Zhang and McCormick also have shown that a series of N-(4'-pyridoxyl)amines, in which several synthetic amines were conjugated at the 4'-position of pyridoxal, are able to enter cells by a process facilitated by the B6 transporter. They also demonstrated the release of these synthetic amines within the cell. Other pyridoxal family members include pyridoxine, pyridoxamine, pyridoxal phosphate, and pyridoxic acid. Pyridoxic acid, niacin, pantothenic acid, biotin, folic acid and ascorbic acid can be conjugated to oligonucleotides using N-hydroxysuccinimide esters that are reactive with aminolinkers located on the oligonucleotide, as described above for retinoic acid.

Other groups for modifying antisense properties include RNA cleaving complexes, pyrenes, metal chelators, porphyrins, alkylators, hybrid intercalator/ligands and photo-crosslinking agents. RNA cleavers include *o*-phenanthroline/Cu complexes and Ru(bipyridine)₃²⁺ complexes. The Ru(bpy)₃²⁺ complexes interact with nucleic acids and cleave nucleic acids photochemically. Metal chelators include EDTA, DTPA, and o-phenanthroline. Alkylators include compounds such as iodoacetamide. Porphyrins include porphine, its substituted forms, and metal complexes. Pyrenes include pyrene and other pyrene-based carboxylic acids that could be conjugated using the similar protocols.

Hybrid intercalator/ligands include the photonuclease/intercalator ligand 6-[[[9-[[6-(4-nitrobenzamido)hexyl]amino]acridin-4-yl]carbonyl]amino]hexanoylpentafluorophenyl ester. This compound has two noteworthy features: an acridine moiety that is an intercalator and a p-nitro benzamido group that is a photonuclease.

Photo-crosslinking agents include aryl azides such as, for example, N-hydroxysucciniimidyl-4-azidobenzoate (HSAB) and N-succinimidyl-6(-4'-azido-2'-nitrophenyl-amino)hexanoate (SANPAH). Aryl azides conjugated to oligonucleotides effect crosslinking with nucleic acids and proteins upon irradiation, They also crosslink with carrier proteins (such as KLH or BSA), raising antibody against the oligonucleotides.

A variety of linking groups can be used to connect the substituents of the invention to nucleosides, nucleotides, and/or oligonucleotides. Certain linking groups, such as n-aminoalkoxy moieties and Ω-aminoalkylamino moieties, are particularly useful for linking steroid molecules or reporter molecules to the 2'-position of a nucleoside. Many linking groups are commercially available, including heterobifunctional and homobifunctional linking moieties available from the Pierce Co. (Rockford, Il). Heterobifunctional and homobifunctional linking moieties are particularly useful in conjunction with the Ω-aminoalkoxy and Ω-aminoalkylamino moieties to form extended linkers that connect peptides and proteins to nucleosides. Other commercially available linking groups are 5'-Amino-Modifier C6 and 3'-Amino-Modifier reagents, both available from Glen Research Corporation (Sterling, VA). 5'-Amino-Modifier C6 is also available from ABI (Applied Biosystems Inc., Foster City, CA) as Aminolink-2, and 3'-Amino-Modifier is also available from Clontech Laboratories Inc. (Palo Alto, CA). A nucleotide analog bearing a linking group pre-attached to the nucleoside is commercially available from Glen Research Corporation under the tradename "Amino-Modifier-dT." This nucleoside-linking group reagent, a uridine derivative having an [N(7-trifluoroacetylaminoheptyl)3-acrylamido] substituent group at the 5 position of the pyrimidine ring, is synthesized generally according to Jablonski, *et al., Nucleic Acid Research* **1986,** *14*, 6115. It is intended that the nucleoside analogs of the invention include adenine nucleosides functionalized with linkers on their N6 purine amino groups, guanine nucleosides functionalized with linkers at their exocyclic N2 purine amino groups, and cytosine nucleosides functionalized with linkers on either their N4 pyrimidine amino groups or 5 pyrimidine positions.

Sequence-specific linked nucleosides of the invention are assembled on a suitable DNA synthesizer utilizing either standard nucleotide precursors or nucleotide precursors that already bear linking moieties. Once synthesis of the sequence-specific linked nucleosides is complete, a substituent can be reacted with the linking moiety. Thus, the invention preferably first builds a desired linked nucleoside sequence by known techniques on a DNA synthesizer. One or more of the linked nucleosides are then functionalized or derivatized with a selected substituent.

PCT/US91/00243, filed January 11, 1991 (equivalent European patent application = EP-A-0604409) and PCT/US91/05720, filed August 12, 1991 (equivalent European patent application = EP-A-0 549 615), disclose that incorporation of, for example, a 2'-O-methyl, 2'-O-ethyl, 2'-O-propyl, 2'-O-allyl, 2'-O-aminoalkyl or 2'-deoxy-2'-fluoro groups on the nucleosides of an oligonucleotide enhance the hybridization properties of the oligonucleotide. These applications also disclose that oligonucleotides containing phosphorothioate backbones have enhanced nuclease stability. The functionalized, linked nucleosides of the invention can be augmented to further include either or both a phosphorothioate backbone or a 2'-O-C₁-C₂₀-alkyl (*e.g.*, 2'-O-methyl, 2'-O-ethyl, 2'-O-propyl), 2'-O-C₂-C₂₀-alkenyl (*e.g.*, 2'-O-allyl), 2'-O-C₂-C₂₀-alkynyl, 2'-S-C₁-C₂₀-alkyl, 2'-S-C₂-C₂₀-alkenyl, 2'-S-C₂-C₂₀-alkynyl, 2'-NH-C₁-C₂₀-alkyl (2'-O-aminoalkyl), 2'-NH-C₂-C₂₀-alkenyl, 2'-NH-C₂-C₂₀-alkynyl or 2'-deoxy-2'-fluoro group. *See, e.g.*, PCT/US93/06807, filed July 20, 1993 (equivalent European patent application = EP-A-0651759), which claims priority from U.S. Application Serial No. 918,362, filed July 23, 1992.

An oligonucleotide possessing an amino group at its 5'-terminus is prepared using a DNA synthesizer and then is reacted with an active ester derivative of the substituent of the invention (*e.g.*, cholic acid). Active ester derivatives are well known to those skilled in the art. Representative active esters include N-hydrosuccinimide esters, tetrafluorophenolic esters, pentafluorophenolic esters and pentachlorophenolic esters. For cholic acid, the reaction of the amino group and the active ester produces an oligonucleotide in which cholic acid is attached to the 5'-position through a linking group. The amino group at the 5'-terminus can be prepared conveniently utilizing the above-noted 5'-Amino-Modifier C6 reagent.

Cholic acid can be attached to a 3'-terminal amino group by reacting a 3'-amino modified controlled pore glass (sold by Clontech Laboratories Inc., Palo Alto, CA), with a cholic acid active ester.

Cholic acid can be attached to both ends of a linked nucleoside sequence by reacting a 3',5'-diamino sequence with the cholic acid active ester. The required oligonucleoside sequence is synthesized utilizing the 3'-Amino-Modifier and the 5'-Amino-Modifier C6 (or Aminolink-2) reagents noted above or by utilizing the above-noted 3'-amino modified controlled pore glass reagent in combination with the 5'-Amino-Modifier C2 (or Aminolink-2) reagents.

In even further embodiments of the invention, an oligonucleoside sequence bearing an aminolinker at the 2'-position of one or more selected nucleosides is prepared using a suitably functionalized nucleotide such as, for example, 5'-dimethoxytrityl-2'-O-(ε-phthalimidylaminopentyl)-2'-deoxyadenosine-3'-N,N-diisopropyl-cyanoethoxy phosphoramidite. *See*, *e.g.*, Manoharan, *et al., Tetrahedron Letters,* **1991,** *34*, 7171 and above-referenced Application Serial Nos. PCT/US91/00243 and PCT/US91/05720 (EP-A-0604409 and EP-A-0549615). The nucleotide or nucleotides are attached to cholic acid or another substituent using an active ester or a thioisocyanate thereof. This approach allows the introduction of a large number of functional groups into an linked nucleoside sequence. Indeed each of the nucleosides can be so substituted.

In further functionalized nucleoside sequences of the invention, the heterocyclic base of one or more nucleosides is linked to a steroid molecule, a reporter molecule, a non-aromatic lipophilic molecule, a reporter enzyme, a peptide, a protein, a water soluble vitamin, a lipid soluble vitamin, an RNA cleaving complex, a metal chelator, a porphyrin, an alkylator, a hybrid photonuclease/intercalator, or an aryl azide photo-crosslinking agent. Utilizing 5'-O-dimethoxytrityl-5-[N(7-trifluoroacetylaminoheptyl)-3-acrylamido]-2'-deoxyuridine 3'-O-(methyl N,N-diisopropyl)phosphoramide, as described by Jablonski, *et al*. above (also commercially available from Glen Research), the desired nucleoside, functionalized to incorporate a linking group on its heterocyclic base, is incorporated into the linked nucleoside sequence using a DNA synthesizer.

Conjugation (linking) of reporter enzymes, peptides, and proteins to linked nucleosides is achieved by conjugation of the enzyme, peptide or protein to the above-described amino linking group on the nucleoside. This can be effected in several ways. A peptide- or protein- functionalized nucleoside of the invention can be prepared by conjugation of the peptide or protein to the nucleoside using EDC/sulto-NHS (*i*.*e*.,1-ethyl-3(3-dimethylaminopropylcarbodiimide/N-hydroxysulfosuccinimide) to conjugate the carboxyl end of the reporter enzyme, peptide, or protein with the amino function of the linking group on the nucleotide. Further, a linked nucleoside sequence of the invention can be prepared using EDC/sulfo-NHS to conjugate a carboxyl group of an aspartic or glutamic acid residue in the reporter enzyme, peptide or protein to the amino function of a linked nucleoside sequence.

A reporter enzyme-, peptide-, protein-functionalized linked nucleoside sequence may be prepared by conjugation of the reporter enzyme, peptide or protein to the nucleoside sequence via a heterobifunctional linker such as m-maleimidobenzoyl-N-hydroxysulfosuccinimide ester (MBS) or succinimidyl 4-(N-maleimidomethyl)cyclohexane-1-carboxylate (SMCC) to link a thiol function on the reporter enzyme, peptide or protein to the amino function of the linking group on nucleoside sequence. By this mechanism, an oligonucleoside-maleimide conjugate is formed by reaction of the amino group of the linker on the linked nucleosides with the MBS or SMCC maleimide linker. The conjugate is then reacted with peptides or proteins having free sulfhydryl groups.

Alternatively, a reporter enzyme-, peptide-, protein-functionalized linked nucleoside sequence may be prepared by conjugation of the peptide or protein to the sequence using a homobifunctional linker such as disuccinimidyl suberate (DSS) to link an amino function on the peptide or protein to the amino group of a linker on the sequence. By this mechanism, an oligonucleoside-succinimidyl conjugate is formed by reaction of the amino group of the linker on the nucleoside sequence with a disuccinimidyl suberate linker. The disuccinimidyl suberate linker couples with the amine linker on the sequence to extend the size of the linker. The extended linker is then reacted with amine groups such as, for example, the amine of lysine or other available N-terminus amines, on reporter enzymes, peptides and proteins.

Additional objects, advantages, and novel features of this invention will become apparent to those skilled in the art upon examination of the following examples, which are not intended to be limiting.

For the following examples, anhydrous dimethylformamide, cholic acid and N-hydroxysuccinimide were purchased from Aldrich Chemical Co. (Milwaukee, WI), ethyl-3-(3-dimethylamino)propylcarbodiimide (EDAC or EDC) was obtained from JBL Scientific (San Luis Obispo, CA) as the free base under the label EDAC or from Pierce (Rockford, Il) under the label EDC, Aminolink-2 was purchased from ABI and 3'-Amino-Modifier, 5'-Amino-Modifier C6 and Amino-Modifier dT reagents were purchased from Glen Research Corporation. NMR Spectra were run on a Varian Unity-400 instrument. Oligonucleotide synthesis were performed on an Applied Biosystems 380 B or 394 DNA synthesizer following standard phosphoramidite protocols using reagents supplied by the manufacturer. When modified phophoramidites were used, a longer coupling time (10-15 min) was employed. HPLC was performed on a Waters 600E instrument equipped with a model 991 detector. Unless otherwise noted, for analytical chromatography the following conditions were employed: Hamilton PRP-1 column (15 x 2.5 cm); solvent A: 50mM TEAA, pH 7.0; solvent B: 45mM TEAA with 80% CH₃CN; flow rate: 1.5ml/min; gradient: 5% B for the first 5 minutes, linear (1%) increase in B every minute thereafter and for preparative purposes: Waters Delta Pak C-4 column; flow rate: 5ml/min; gradient: 5% B for the first 10 minutes, linear 1% increase for every minute thereafter.

All oligonucleotide sequences are listed in a standard 5' to 3' order from left to right.

### EXAMPLE 1

### Cholic Acid N-Hydroxysuccinimide Ester (Compound 1)

Anhydrous DMF (150ml) was added to a mixture of cholic acid (4.09g, 15mmol) and N-hydroxysuccinimide (5.25g, 45 mmol). The mixture was stirred in the presence of nitrogen. EDAC (4ml, 25mmol) was then added and this mixture was then stirred overnight. The solution was then evaporated to a gum and partitioned between 1:1 ethyl acetate and 4% NaHCO₃ solution (pH 7.9) (100ml each). The organic layer was washed with saturated NaCl solution, dried over anhydrous Na₂SO₄ and evaporated to yield the title compound as a pale yellow foam (4.6g, 91%). ¹³C NMR (DMSO-d₆) δ 12.27, 16.71, 22.58, 22.80, 25.42, 26.19, 27.20, 28.49, 30.41, 30.56, 34.36, 34.82, 34.82, 35.31, 39.09, 39.09, 41.38, 41.53, 45.84, 46.05, 66.25, 70.45, 71.03, 169.28 and 170.16.

### EXAMPLE 2

### Heterocyclic Base Cholic Acid End-Labeled Oligodeoxynucleotide

An oligonucleotide of the sequence: Oligomer 1:    TTG CTT* CCA TCT TCC TCG TC wherein T* represents a nucleotide functionalized to included a cholic acid linked via a linker to the heterocyclic base of a 2'-deoxyuridine nucleotide was prepared in a 1 µmol scale. Oligomer 1 is useful as a human papilloma virus (HPV) antisense oligonucleotide.

### A. Preparation of Intermediate Linker

The linker of the structure -CH=CH-C(O)-NH-(CH₂)₆-NH-C(O)-CF₃ was introduced via a suitable protected and activated 2'-deoxyuridine phosphoramide intermediate obtained from-Glen Research Corporation as Amino-Modifier-dT. The oligonucleotide bearing the linker thereon was deprotected and purified by HPLC.

### B. Preparation of Cholic Acid Functionalized Oligonucleotide

An aliquot of the linker bearing oligonucleotide of Example 2-A (approximately 100 O.D. units, 550 nmols) was dissolved in 500 µl of 0.2M NaHCO₃ buffer and to this solution cholic acid N-hydroxysuccinimide ester (Compound 1, 75 mg, 149 µmols) was added and heated at 45°C overnight. It was then passed through a Sephadex® G-25 (1.0 x 25 cm) column. Concentration of the oligonucleotide fractions to 2 ml followed by HPLC purification yielded the desired conjugate wherein cholic acid is internally linked at C-5 of the heterocyclic base.

### EXAMPLE 3

### 5'-Terminus Cholic Acid End-Labeled Oligodeoxynucleotide

A phosphorothioate oligonucleotide having cholic acid attached to its 5'-terminus of the oligonucleotide sequence: 
Oligomer 2:
5'-CHA-C_sT_sG_s T_sC_sT_s C_sC_sA_s T_sC_sT_s T_sC_sA_s C_sT
 wherein CHA represents cholic acid and the subscript "s" represents a phosphorothioate inter-nucleotide backbone linkage was prepared.

### A. Preparation of Intermediate Linker

The oligonucleotide sequence having a 5'-terminus amino group was synthesized on a 3 x 1.0 µmol scale in the standard manner on the DNA synthesizer utilizing phosphoramidite methodology. The phosphorothioate intra-nucleotide backbone was formed using a phosphorothioate reagent (Beaucage reagent, *i.e.,* 3H-1,2-benzodithioate-3-one 1,1-dioxide; *see*, Radhakrishnan, *et al., J. Am. Chem. Soc.* **1990,** *112*, 1253). The Aminolink-2 reagent was used at the last step of the oligonucleotide synthesis. Deprotection with concentrated NH₄OH for 16 hrs at 55°C yielded the 5'-aminolinker-oligonucleotide.

### B. Preparation of Cholic Acid Functionalized Oligonucleotide

The crude 5'-aminolinker-oligonucleotide of Example 3-A (100 O.D. units, approximately 600 nmols based on the calculated extinction coefficient of 1.6756 x 10⁵ at 260 nm) was dissolved in freshly prepared NaHCO₃ buffer (500 µl, 0.2M, pH 8.1) and treated with a solution of cholic acid N-hydroxysuccinimide ester (Compound 1, 75 mg, 149 µmols) dissolved in 200 µl of DMF. The reaction mixture was heated at 45°C overnight. It was then passed through a Sephadex® G-25 (1.0 x 25 cm) column. Concentration of the oligonucleotide fractions to 2 ml followed by HPLC purification yielded 54 OD units of the desired conjugate (54% yield). HPLC retention times were: 37.42 for the unreacted oligonucleotide and any failure sequences produced during oligonucleotide synthesis and 54.20 for the final product.

### EXAMPLE 4

### 3'-Terminus Cholic Acid End Labeled Oligodeoxynucleotide

### A. 3'-Terminus Cholic Acid Oligonucleotide

A phosphorothioate oligonucleotide having cholic acid attached to its 3'-terminus of the oligonucleotide sequence: 
Oligomer 3:
CₛTₛGₛ TₛCₛTₛ CₛCₛAₛ TₛCₛCₛ TₛCₛTₛ TₛCₛAₛ CₛT 3′-CHA
 wherein CHA represents cholic acid and the subscript "s" represents a phosphorothioate inter-nucleotide backbone linkage was prepared.

### 1. Preparation of Intermediate Linker

The oligonucleotide sequence having a 3'-terminus amino group was synthesized using 3'-amino modifier controlled pore glass (CPG) from Clontech Laboratories (Palo Alto, CA) as the solid support. The above-noted Beaucage reagent was utilized to form the phosphorothioate inter-nucleotide backbone. The synthesis was conducted in a "Trityl-Off" mode. The resultant solid support was deprotected with concentrated NH₄OH for 16 hrs at 55°C. Purification on a Sephadex® G-25 column yielded a 3'-amino functionalized phosphorothioate oligonucleotide of the specified oligonucleotide sequence.

### 2. Preparation of Cholic Acid Functionalized Oligonucleotide

The crude oligonucleotide of Example 4-A-1 (50 O.D. units, approximately 300 nmols) was reacted with cholic acid N-hydroxysuccinimide ester (Compound 1, 40 mg) as per the procedure of Example 3. HPLC retention times were 37.45 for the starting oligonucleotide material and 51.68 for the product.

### B. 3'-Terminus Cholic Acid Oligonucleotide

A phosphorothioate oligonucleotide having cholic acid attached to its 3'-terminus and of the oligonucleotide sequence: 
Oligomer 4:
TₛGₛGₛ GₛAₛGₛ CₛCₛAₛ TₛAₛGₛ CₛGₛAₛ GₛGₛC 3'-CHA
 wherein CHA represents cholic acid and the subscript "s" represents a phosphorothioate inter-nucleotide backbone linkage was prepared in the same manner as for the oligonucleotide of Example 4-A-2.

### EXAMPLE 5

### 3'-Terminus Cholic Acid & 5'-Terminus Cholic Acid Di-End-Labeled Oligodeoxynucleotide

A phosphorothioate oligonucleotide having cholic acid attached to both of the 3'-terminus and the 5'-terminus of the oligonucleotide sequence: 
Oligomer 5:
5'-CHA CₛTₛGₛ TₛCₛTₛ CₛCₛAₛ TₛCₛTₛ TₛCₛAₛ CₛT 3'-CHA
 wherein CHA represents cholic acid and the subscript "s" represents a phosphorothioate inter-nucleotide backbone linkage was synthesized on a 3 x 1.0 µmol scale. Oligomer 5 has the same sequence as Oligomer 1 except for the cholic acid functionalization.

### A. Preparation of Intermediate Linker

The oligonucleotide synthesis was conducted using a 3'-amino modified controlled pore glass (CPG) from Clontech Laboratories as the solid support. Oligonucleotide synthesis was conducted utilizing phosphoramidite synthetic methodology and the Beaucage reagent to form a phosphorothioate inter-nucleotide backbone as per Example 4 above. Upon completion of the basic sequence and while still on the DNA synthesizer, Aminolink-2 reagent was used to introduce a 5'-terminus amino functionality on to the oligonucleotide. Deprotection with concentrated ammonium hydroxide and purification on a Sephadex® G-25 column yielded 3',5'-diaminolinker oligonucleotide.

### B. Preparation of Cholic Acid Functionalized Oligonucleotide

The crude di-aminolinker oligonucleotide (50 O.D. units, approximately 300 nmols, based on the calculated extinction coefficient of 1.6756 x 10⁵ at 260 nm) was dissolved in freshly prepared NaHCO₃ buffer (500 µl, 0.2M, pH 8.1) and treated with a solution of cholic acid N-hydroxysuccinimide ester (Compound 1, 50 mg, 98.6 µmols) dissolved in 200 µl of DMF. The reaction mixture was heated at 45°C overnight. It was then passed through a Sephadex® G-25 (1.0 x 25 cm) column. The oligonucleotide fractions were concentrated to 2 ml and purified by reverse phase HPLC. Retention times were 37.76 for unreacted oligonucleotide, 51.65 for 3'-cholic acid conjugated oligonucleotide, 54.34 for 5'-cholic acid conjugated oligonucleotide and 58.75 for 3',5'-di-cholic acid conjugated oligonucleotide. The 58.75 min. product was desalted on a Sephadex® G-25 column to yield 11 O.D units (22%) of the desired product.

### EXAMPLE 6

### 3'-Terminus Cholic Acid or 5'-Terminus Cholic Acid Functionalized, 2'-O-Methyl Derivatized Oligodeoxynucleotides

Phosphorothioate oligonucleotides having cholic acid attached to either the 3'-terminus end or the 5'-terminus end of the oligonucleotide sequence and further being uniformly functionalized to include a 2'-O-methyl group on each of the nucleotides of the oligonucleotide were synthesized. The following oligonucleotides having uniform 2'-O-methyl substitutions were synthesized: Oligomer 6:    5′-CHA CCC AGG CUC AGA 3′; Oligomer 7:    5′ CCC AGG CUC AGA 3′-CHA; and

Oligomer 8:    5'-CHA GAG CUC CCA GGC 3'.

### A. Preparation of Intermediate Linker

Synthesis of the intermediate 5' or 3'-aminolinker oligonucleotide was conducted utilizing 2'-O-methyl phosphoramidite nucleotides available from Chemgenes Inc. (Needham, MA) and phosphoramidite chemistry as per Examples 3 and 4 above, respectively. Each of the intermediate oligonucleotides were deprotected in concentrated NH₄OH, evaporated and de-salted on a Sephadex® G-25 column.

### B. Preparation of Cholic Acid Functionalized oligonucleotide

The resultant crude oligonucleotides from Example 6-A-1 (from 30 to 40 O.D. units, 250-350 nmols based on calculated extinction coefficients of 1.1797 x 10⁵, 1.1777 x 10⁵ and 1.1481 x 10⁵ at 260 nm, respectively for Oligomers 6, 7 and 8) were dried and dissolved in 250 µl of 0.2M NaHCO₃ buffer and treated with a solution of cholic acid N-hydroxysuccinimide ester (Compound 1, from 30 to 40 mg, 60 to 80 µmols) dissolved in 500 µl of 0.2 M NaHCO₃ buffer and 200 µl DMF and heated between 40-45°C for 12-24 hrs. The reaction mixtures were evaporated and dissolved in 2 ml of water and washed with 3 x 2 ml of ethyl acetate. The resultant aqueous solutions were purified by reverse phase HPLC. For Oligomer 6 the HPLC retention times were 34.65 for the starting oligonucleotide material and 58.75 for the product; for Oligomer 7 the HPLC retention times were 37.23 for the starting oligonucleotide material and 55.32 for the product; and for Oligomer 8 the HPLC retention times were 34.99 for the starting oligonucleotide material and 56.98 for the product. The products were evaporated and desalted on a Sephadex® G-25 column. The yield averaged about 20% in each case.

### EXAMPLE 7

### Oligonucleotides Having 2'-Protected-Amine Terminating Linking Group

### A. Preparation of 5'-Dimethoxytrityl-2'-(O-Pentyl-N-phthalimido)-2'-Deoxyadenosine Phosphoramidite (Compound 2)

To introduce a functionalization at the 2' position of nucleotides within desired oligonucleotide sequences, 5'-Dimethoxytrityl-2'-(O-pentyl-N-phthalimido)-2'-deoxyadenosine phosphoramidite (Compound 2) was utilized to provide a linking group attached to the 2' position of nucleotide components of an oligonucleotide. Compound 2 was synthesized as per the procedures of patent application PCT/US91/00243, identified above starting from adenosine. Briefly this procedure treats adenosine with NaH in DMF followed by treatment with N-(5-bromopentyl)phthalimide. Further treatment with (CH₃)₃SiCl, Ph-C(O)-Cl and NH₄OH yields N6-benzyl protected 2'-pentyl-N-phthalimido functionalized adenosine. Treatment with DIPA and CH₂Cl₂ adds a DMT blocking group at the 5' position. Finally phosphitylation gives the desired phosphoramidite compound, Compound 2. Compound 2 was utilized in the DNA synthesizer as a 0.09M solution in anhydrous CH₃CN. Oligonucleotide synthesis was carried out in either an ABI 390B or 394 synthesizer employing the standard synthesis cycles with an extended coupling time of 10 minutes during coupling of Compound 2 into the oligonucleotide sequence. Coupling efficiency of > 98% was observed for Compound 2 coupling.

### B. 2'-Protected-Amine Linking Group Containing Phosphodiester Oligonucleotides

The following oligonucleotides having phosphodiester inter-nucleotide linkages were synthesized: Oligomer 9:    5′ TA*G 3′; Oligomer 10:   5′ CCA* G 3′; Oligomer 11:   5′ GGC TGA* CTG CG 3′; Oligomer 12: CTG TCT CCA* TCC TCT TCA CT; and Oligomer 13:   CTG TCT CCA* TCC TCT TCA* CT wherein A* represents a nucleotide functionalized to incorporate a pentyl-N-phthalimido functionality. Oligomers 12 and 13 are antisense compounds to the E2 region of the bovine papilloma virus-1 (BPV-1). Oligomers 12 and 13 have the same sequence as Oligomer 3 except for the 2' modification. The oligonucleotides were synthesized in either a 10 µmol scale or a 3 x 1 µmol scale in the "Trityl-On" mode. Standard deprotection conditions (30% NH₄OH, 55°C, 24 hr) were employed. The oligonucleotides were purified by reverse phase HPLC (Waters Delta-Pak C₄ 15 µm, 300A, 25x100 mm column equipped with a guard column of the same material). They were detritylated and further purified by size exclusion using a Sephadex® G-25 column. NMR analyses by both proton and phosphorus NMR confirmed the expected structure for the Oligomers 9 and 10.

### C. 2'-Protected-Amine Linking Group Containing Phosphorothioate Oligonucleotides

The following oligonucleotides having phosphorothioate inter-nucleotide linkages were synthesized: 
Oligomer 14:
TsTsGs CsTsTs CsCsA*s TsCsTs TsCsCs TsCsGs TsC;
 
Oligomer 15:
TsGsGs GsAsGs CsCsAs TsAsGs CsGsA*s GsGsC;
 and 
Oligomer 16:
TsGsGs GsA*sGs CsCsA*s TsA*sGs CsGsA*s GsGsC
 wherein A* represents a nucleotide functionalized to incorporate a pentyl-N-phthalimido functionality and the subscript "s" represents a phosphorothioate inter-nucleotide backbone linkage. Oligomer 14 is an antisense compound directed to the E2 region of the bovine papilloma virus-1 (BPV-1). Oligomers 15 and 16 are antisense compounds to intercellular adhesion molecule (ICAM). Oligomer 14 has the same sequence as Oligomer 3 except for the 2' modification whereas Oligomers 15 and 16 have the same sequence as Oligomer 4 except for the 2' modification. These oligonucleotides were synthesized as per the method of Example 7-B except during the synthesis, for oxidation of the phosphite moieties, the Beaucage reagent (see Example 3 above) was used as a 0.24 M solution in anhydrous CH₃CN solvent. The oligonucleotides were synthesized in the "Trityl-On" mode and purified by reverse phase HPLC utilizing the purification procedure of Example 7-B.

### D. 2'-O-Methyl Derivatized, 2'-Protected-Amine Linking Group Containing RNA Oligonucleotides

The following oligonucleotides having 2'-O-methyl groups on each nucleotide not functionalized with a 2'-protected amine functionalization were synthesized: Oligomer 17: CCA A*GC CUC AGA; and Oligomer 18:   CCA GGC UCA GA*T wherein A* represents a nucleotide functionalized to incorporate a pentyl-N-phthalimido functionality and where the remaining nucleotides except the 3'-terminus nucleotide are each 2'-O-methyl derivatized nucleotides. The 3'-terminus nucleotide in both Oligomers 17 and 18 is a 2'-deoxy nucleotide. Both Oligomers 17 and 18 are antisense compounds to the HIV-1 TAR region. The oligonucleotides were synthesized as per the method of Example 6 utilizing Compound 2 for introduction of the nucleotides containing the pentyl-N-phthalimido functionality and appropriate 2-O-methyl phosphoramidite nucleotides from Chemgenes Inc. (Needham, MA) for the remaining RNA nucleotides. The 3'-terminus terminal 2'-deoxy nucleotides were standard phosphoamidites utilized for the DNA synthesizer. The oligonucleotides were deprotected and purified as per the method of Example 7-B.

### EXAMPLE 8

### Functionalization Of Oligonucleotides At the 2' Position

### A. Functionalization with Biotin

### 1. Single Site Modification

About 10 O.D. units (A₂₆₀) of Oligomer 12 (see Example 7) (approximately 60 nmols based on the calculated extinction coefficient of 1.6756 x 10⁵) was dried in a microfuge tube. The oligonucleotide was dissolved in 200 µl of 0.2 M NaHCO₃ buffer and D-biotin-N-hydroxysuccinimide ester (2.5 mg, 7.3 µmols) (Sigma, St. Louis, MO) was added followed by 40 µl DMF. The solution was let stand overnight. The solution was applied to a Sephadex® G-25 column (0.7 x 15 cm) and-the oligonucleotide fractions were combined. Analytical HPLC showed nearly 85% conversion to the product. The product was purified by HPLC (Waters 600E with 991 detector, Hamilton PRP-1 column 0.7 x 15 cm; solvent A: 50 mM TEAA pH 7.0; B: 45 mM TEAA with 80% acetonitrile: 1.5 ml flow rate: Gradient: 5% B for first 5 mins., linear (1%) increase in B every minute thereafter) and further desalted on Sephadex® G-25 to give the oligonucleotide: Oligomer 19: CTG TCT CCA* TCC TCT TCA CT wherein A* represents a nucleotide functionalized to incorporate a biotin functionality linked via a 2'-O-pentylamino linking group to the 2' position of the designated nucleotide. HPLC retention times are shown in Table 1 below.

### 2. Multiple Site Modification

About 10 O.D. units (A₂₆₀) of Oligomer 13 (see Example 7, approximately 60 nmols) was treated utilizing the method of Example 8-A-1 with D-biotin-N-hydroxysuccinimide ester (5 mg) in 300 µl of 0.2 M NaHCO₃ buffer/ 50 µl DMF. Analytical HPLC showed 65% of double labeled oligonucleotide product and 30% of single labeled products (from the two available reactive sites). HPLC and Sephadex® G-25 purification gave the oligonucleotide: Oligomer 20: CTG TCT CCA* TCC TCT TCA* CT wherein A* represents nucleotides functionalized to incorporate a biotin functionality linked via a 2'-O-pentylamino linking group to the 2' position of the designated nucleotide. HPLC retention times for this product (and its accompanying singly labeled products) are shown in Table 1 below.

### B. Functionalization with Fluorescein

### 1. Single Site Modification

A 1M Na₂CO₃/1M NaHCO₃ buffer (pH 9.0) was prepared by adding 1M NaHCO₃ to 1 M Na₂CO₃. 200 µl of this buffer was added to 10 O.D. units of Oligomer 12 (see Example 7) in a microfuge tube. 10 mg of fluorescein-isocyanate in 500 µl DMF was added to give a 0.05 M solution. 100 µl of the fluorescein solution was added to the oligonucleotide solution in the microfuge tube. The tube was covered with aluminum foil and let stand overnight. The reaction mixture was applied to a Sephadex® G-25 column (0.7 x 20 cm) that had been equilibrated with 25% (v/v) ethyl alcohol in water. The column was eluted with the same solvent. Product migration could be seen as a yellow band well separated from dark yellow band of the excess fluorescein reagent. The fractions showing absorption at 260 nm and 485 nm were combined and purified by HPLC as per the purification procedure of Example 8-A-1. Analytical HPLC indicated 81% of the desired doubly functionalized oligonucleotide. The product was lyophilized and desalted on Sephadex® to give the oligonucleotide: Oligomer 21:   CTG TCT CCA* TCC TCT TCA CT wherein A* represents a nucleotide functionalized to incorporate a fluorescein functionality linked via a 2'-O-pentyl-amino linking group to the 2' position of the designated nucleotide. HPLC retention times are shown in Table 1 below.

### 2. Multiple Site Modification

10 O.D. units (A₂₆₀) of Oligomer 13 (from Example 7) was dissolved in 300 µl of the 1M Na₂HCO₃/ 1M Na₂CO₂ buffer of Example 8-B-1 and 200 µl of the fluorescein-isothiocyanate stock solution of Example 8-B-1 was added. The resulting solution was treated as per Example 8-B-1. Analytical HPLC indicated 61% of doubly labeled product and 38% of singly labeled products. Work up of the reaction gave the oligonucleotide: Oligomer 22: CTG TCT CCA* TCC TCT TCA* CT wherein A* represents nucleotides functionalized to incorporate a fluorescein functionality linked via a 2'-O-pentyl-amino linking group to the 2' position of the designated nucleotide. HPLC retention times are shown in Table 1 below.

### C. Functionalization with Cholic Acid

### 1. Single Site Modification

10 O.D. units (A₂₆₀) of Oligomer 12 (see Example 7) was treated with cholic acid-NHS ester (Compound 1, 5 mg, 9.9 µmols) in 200 µl of 0.2 M NaHCO₃ buffer/ 40 µl DMF. The reaction mixture was heated for 16 hrs at 45°C. The product was isolated as per the method of Example 8-A-1. Analytical HPLC indicated > 85% product formation. Work up of the reaction gave the oligonucleotide: Oligomer 23: CTG TCT CCA TCC TCT TCA CT wherein A* represents a nucleotide functionalized to incorporate a cholic acid functionality linked via a 2'-O-pentyl-amino linking group to the 2' position of the designated nucleotide. HPLC retention times are shown in Table 1 below.

### 2. Multiple Site Modification

10 O.D. units (A₂₆₀) of Oligomer 13 (see Example 7) was treated with cholic acid-NHS ester (Compound 1, 10 mg, 19.8 µmols) in 300 µl of 0.2 M NaHCO₃ buffer/ 50 µl DMF. The reaction mixture was heated for 16 hrs at 45°C. The product was isolated as per the method of Example 8-A-1. Analytical HPLC revealed 58% doubly labeled product, 17% of a first singly labeled product and 24% of a second singly labeled product. Work up as per Example 8-A-1 gave the oligonucleotide: Oligomer 24: CTG TCT CCA* TCC TCT TCA* CT wherein A* represents nucleotides functionalized to incorporate a cholic acid functionality linked via a 2'-O-pentyl-amino linking group to the 2' position of the designated nucleotide. HPLC retention times are shown in Table 1 below.

### D. Functionalization with Digoxigenin

### 1. Single Site Modification

10 O.D. units (A₂₆₀) of Oligomer 12 (see Example 7) was treated with digoxigenin-3-O-methylcarbonyl-e-aminocaproic N-hydroxy succinimide ester (Boehringer Mannheim Corporation, Indianapolis, IN) in 200 µl of 0.1 M borate pH 8.3 buffer/ 40 µl DMF. The reaction mixture was let stand overnight. The product was isolated as per the method of Example 8-A-1. Work up of the reaction gave the oligonucleotide: Oligomer 25:   CTG TCT CCA* TCC TCT TCA CT wherein A* represents a nucleotide functionalized to incorporate a digoxigenin functionality linked via a 2'-O-pentyl-amino linking group to the 2' position of the designated nucleotide. HPLC retention times are shown in Table 1 below.

### 2. Multiple Site Modification

10 O.D. units (A₂₆₀) of Oligomer 13 (see Example 7) was treated with digoxigenin-3-O-methylcarbonyl-e-aminocaproic N-hydroxy succinimide ester (Boehringer Mannheim Corporation, Indianapolis, IN) in 300 µl of 0.1 M borate pH 8.3 buffer / 50 µl DMF. The reaction mixture was let stand overnight. The product was isolated as per the method of Example 8-A-1. Work up as per Example 8-A-1 gave the oligonucleotide: Oligomer 26: CTG TCT CCA* TCC TCT TCA* CT wherein A* represents nucleotides functionalized to incorporate a cholic acid functionality linked via a 2'-O-pentyl-amino linking group to the 2' position of the designated nucleotide. HPLC retention times are shown in Table 1 below.

**TABLE 1**

| **HPLC Retention Times Of Oligonucleotides Functionalized At 2' Position** | | | |
|---|---|---|---|
| | Retention Time Minutes | | |
| Oligomer | Mono Substitution | Multiple Substitution | |
| Oligomer 12¹ | 21.78 | | |
| Oligomer 13¹ | | 22.50 | |
| Oligomer 19² | 23.58 | | |
| Oligomer 20³ | | 24.16^{a} | 25.19^{b} |
| Oligomer 21³ | 26.65 | | |
| Oligomer 22³ | | 26.99^{a} | 29.33^{b} |
| | | 27.55^{a} | |
| Oligomer 23⁴ | 30.10 | | |
| Oligomer 24⁴ | | 30.38^{a} | 37.00^{b} |
| | | 32.22^{a} | |
| Oligomer 25⁵ | 28.06 | | |
| Oligomer 26⁵ | | 28.14^{a} | 33.32^{b} |
| | | 29.24^{a} | |

| | | | |
|---|---|---|---|
| Conditions: Waters 600E with 991 detector, Hamilton PRP-1 column 0.7 x 15 cm; solvent A: 50 mM TEAA pH 7.0; B: 45 mM TEAA with 80% acetonitrile: 1.5 ml flow rate: Gradient: 5% B for first 5 mins., linear (1%) increase in B every minute thereafter; ^{a} Mono conjugated minor product; | | | |
| ^{b} Doubly conjugated major product; | | | |
| ¹ Parent Oligonucleotide - no 2' functionalization; | | | |
| ² 2' Biotin functionalization; | | | |
| ³ 2' Fluorescein functionalization; | | | |
| ⁴ 2' Cholic Acid functionalization; and | | | |
| ⁵ 2' Digoxigenin functionalization. | | | |

### EXAMPLE 9

### Functionalization Of Oligonucleotide At the 2' Position with Reporter Enzymes, Peptides and Proteins

### A. Use of Heterobifunctional Linker

### 1. Synthesis of Oligonucleotide-Maleimide Conjugate

Oligomer 12 (Example 7) (100 O.D. units, 600 nmols) is lyophilized in a 5 ml pear-shaped flask. Sulfo-SMCC reagent, Pierce Chemical Co. (Rockford, Il) (16 mg, 46 µmols) is dissolved in phosphate buffer (800 µl, 0.1M, pH 7.0) and added to the oligonucleotide bearing flask. An additional 200 µl of buffer are used to wash the reagent and transfer it to the oligonucleotide flask. The contents of the flask are stirred overnight and loaded on to a Sephadex® G-25 column (1 x 40 cm) equipped with a fraction collector. The oligonucleotide-maleimide conjugate containing fractions are collected and tested by analytical HPLC for separation from other NHS type products.

### 2. Synthesis of Oligonucleotide-Peptide Conjugate

An aliquot of the oligonucleotide-maleimide conjugate of Example 9-A-1 (about 50 O.D. units, 300 nmols) is lyophilized in a microfuge tube. SV40 peptide (pro-asp-lys-lys-arg-lys-cys)(2.5 mg, about 2.5 µmols) is taken up in phosphate buffer (800 µl, 0.1 M, pH 7.0) and added to the oligonucleotide-maleimide conjugate containing tube. The contents of the tube are stirred overnight under an argon atmosphere. The reaction mixture is passed through a Sephadex® G-25 column and the oligonucleotide-peptide conjugate fractions are identified by HPLC. Isolation of the product from product-bearing fractions via HPLC and desalting on Sephadex® G-25 will yield an oligonucleotide of the sequence: Oligomer 27: CTG TCT CCA* TCC TCT TCA CT wherein A* represents a nucleotide functionalized to incorporate a SV40 peptide functionality linked via a 2'-O-pentyl-amino-sulfo-SMCC (sulfosuccininidyl-4-(N-maleimidomethyl)cyclohexane-1-carboxylate) linking group to the 2' position of the designated nucleotide.

### B. Use of Homobifunctional Linker

### 1. Synthesis of Oligonucleotide-Disuccinimidyl Suberate Conjugate

An aliquot (10 O.D. units, 60 nmols) of Oligomer 12 (Example 7) is evaporated to dryness and is dissolved in freshly prepared 0.1 M NaHCO₃/50 mM EDTA (100 µl, pH 8.25). The solution is then treated with a solution of DSS, Pierce Chemical Co. (Rockford, Il) (2.6 mg, 7 µmol) in 200 µl DMSO. The solution is stored at room temperature for 15 minutes and then immediately applied to a Sephadex® G-25 column (1 x 40 cm) that is previously packed and washed with water at 4°C. The oligonucleotide fractions are combined immediately in a 25 ml pear-shaped flask and are rapidly frozen in dry ice/isopropyl alcohol and lyophilized to a powder.

### 2. Synthesis of Oligonucleotide-Protein Conjugate

A solution of calf intestinal alkaline phosphatase (Boehringer Mannheim) (20.6 mg, 2.06 ml, 147 nmol) is spun at 4°C in a Centricon® microconcentrator at 6000 rpm until the volume is less than 50 µl. It is then redissolved in 1 ml of cold Tris buffer (pH 8.5, 0.1M containing 0.1 NaCl and 0.05 M MgCl₂) and concentrated twice more. Finally the concentrate is dissolved in 400 µl of the same buffer. This solution is added to the activated oligonucleotide from Example 9-B-1 and the solution stored for 18 hrs at room temp. The product is diluted to approximately 30 ml and applied to a Sephadex® G-25 column (1 x 20 cm, chloride form) maintained at 4°C. The column is eluted with 50 nM Tris-Cl pH 8.5 until the UV absorbance of the fractions eluted reach near zero values. The column is then eluted with a NaCl salt gradient 0.05 M to 0.75 M (150 ml each). The different peaks are assayed for both oligonucleotide and alkaline phosphatase activity and the product bearing fractions are combined. Typically the first peak will be excess enzyme, the second peak the oligonucleotide-protein conjugate and the third peak unreacted oligonucleotide. Isolation of the product from the product-bearing fractions via HPLC and desalting on Sephadex® G-25 will yield an oligonucleotide of the sequence: Oligomer 28: CTG TCT CCA* TCC TCT TCA CT wherein A* represents a nucleotide functionalized to incorporate an alkaline phosphatase functionality linked via an 2'-O-pentyl-amino-sulfo-SMCC (sulfosuccinimidyl-4-(N-maleimidomethyl)cyclohexane-1-carboxylate) linking group to the 2' position of the designated nucleotide.

### EXAMPLE 10

### Heterocyclic Base Peptide-Conjugated Oligonucleotides

Utilizing the method of Example 9-A-1 the intermediate amino linker oligonucleotide of Example 2-A is reacted with sulfo-SMCC reagent. The isolated oligonucleotide-maleimide conjugate is then further reacted with SV40 peptide as per Example 9-A-2. This will give an oligonucleotide of the structure: Oligomer 29: TTG CTT* CCA TCT TCC TCG TC wherein T* represents a nucleotide functionalized to include a peptide linked via an extended linker to the heterocyclic base of a 2'-deoxyuridine nucleotide.

### EXAMPLE 11

### 3'-Terminus Protein-Conjugated Oligonucleotides

Utilizing the method of Example 9-B-1 the 2'-O-methyl derivatized intermediate amino linker oligonucleotide of Example 6-A is reacted with DSS reagent. The isolated oligonucleotide-disuccinimidyl suberate conjugate is then further reacted with a lysine containing Nuclease RNase H using the method of Example 9-B-2. This will give an oligonucleotide of the structure: Oligomer 30:    CsCsCs AsGsGs CsUsCs AsGsA-3′-protein wherein protein represents RNase H, the subscript "s" represents a phosphorothioate inter-nucleotide backbone linkage and each of the nucleotides of the oligonucleotide includes a 2'-O-methyl group thereon.

### EXAMPLE 12

### 5'-Terminus Protein-Conjugated 2'-O-Methyl Derivatized oligonucleotides

Utilizing the method of Example 9-B-1 the 2'-O-methyl derivatized intermediate amino linker oligonucleotide of Example 6-A (Oligomer 6) is reacted with DSS reagent. The isolated oligonucleotide-disuccinimidyl suberate conjugate is then further reacted with a lysine containing Staphylococcal Nuclease using the method of Example 9-B-2. This will give an oligonucleotide of the structure: Oligomer 31:    5'-protein-CₛCₛCₛ AₛGₛGₛ CₛUₛCₛ AₛGₛA 3' wherein protein represents Staphylococcal Nuclease, the subscript "s" represents a phosphorothioate inter-nucleotide backbone linkage and each of the nucleotides of the oligonucleotide includes a 2'-O-methyl group thereon.

### PROCEDURE A

### Confirmation of Structure of Functionalized Oligonucleotides Containing A Tethered 2'-Amino Moiety

Oligonucleotides of the invention were digested with snake venom phosphodiesterase and calf-intestine alkaline phosphatase to their individual nucleosides. After digestion, the nucleoside composition was analyzed by HPLC. The HPLC analysis established that functionalized nucleotide compounds having the tethered 2'-amino moiety thereon were correctly incorporated into the oligonucleotide.

Snake venom phosphodiesterase [Boehringer-Mannheim cat. #108260, 1 mg (1.5 units)/0.5 ml] and alkaline phosphatase from calf intestine (1 unit/microliter, Boehringer-Mannheim cat. # 713023) in Tris-HCl buffer (pH 7.2, 50 mM) were used to digest the oligonucleotides to their component nucleosides. To 0.5 O.D. units of oligonucleotide in 50 µl buffer (nearly 40 µM final concentration for a 20 mer) was added 5 µl of snake venom phosphodiesterase (nearly 0.3 units/ml, final concentration) and 10 µl of alkaline phosphatase (app. 150 units/ml, final concentration). The reaction mixture was incubated at 37°C for 3 hours. Following incubation, the reaction mixture was analyzed by HPLC using a reverse phase analytical column (app. 30 x 2.5 cm); solvent A: 50 mM TEAA pH 7; solvent B: acetonitrile; gradient 100% for 10 mins, then 5% B for 15 mins, then 10% B and then wash. The results of these digestion are shown in Table 2 for representative oligonucleotides.

**TABLE 2**

| **OLIGONUCLEOTIDE ANALYSIS VIA ENZYMATIC DIGESTION** | | | | | | |
|---|---|---|---|---|---|---|
| | Observed Ratios** | | | | | |
| Oligomer | Abs. max. | 267 C | 252 G | 267 T | 260 A* | A |
| Oligomer 10 | | 2 | 1 | | 1 | |
| Oligomer 11 | | 3 | 5 | 2 | 1 | |
| Oligomer 12 | | 9 | 1 | 8 | 1 | 1 |
| Oligomer 13 | | 9 | 1 | 8 | 2 | |

| | | | | | | |
|---|---|---|---|---|---|---|
| * Nucleoside having 2'-O-linker attached thereto; and | | | | | | |
| ** Corrected to whole numbers. | | | | | | |

As is evident from Table 2, the correct nucleoside ratios are observed for the component nucleotides of the test oligonucleotides.

### PROCEDURE B

### Determination of Melting Temperatures (Tm's) of Cholic Acid Oligonucleotide Conjugates

The relative ability of oligonucleotides to bind to their complementary strand is compared by determining the melting temperature of the hybridization complex of the oligonucleotide and its complementary strand. The melting temperature (Tm), a characteristic physical property of double helices, denotes the temperature in degrees centigrade at which 50% helical versus coil (un-hybridized) forms are present. Tm is measured by using the UV spectrum to determine the formation and breakdown (melting) of hybridization. Base stacking, which occurs during hybridization, is accompanied by a reduction in UV absorption (hypochromicity). Consequently a reduction in UV absorption indicates a higher Tₘ. The higher the Tm, the greater the strength of the binding of the strands. Non-Watson-Crick base pairing has a strong destabilizing effect on the Tm. Consequently, absolute fidelity of base pairing is necessary to have optimal binding of an antisense oligonucleotide to its targeted RNA.

### 1. Terminal End Conjugates

### a. Synthesis

A series of oligonucleotides were synthesized utilizing standard synthetic procedures (for un-functionalized oligonucleotides) or the procedure of Example 3-A above for oligonucleotides having a 5'-terminus amino linker bearing oligonucleotide or the procedure of Example 3-B for 5'-terminus cholic acid-bearing oligonucleotides. Each of the oligonucleotides had the following 5-LO antisense sequence: 5' TCC AGG TGT CCG CAT C 3'. The nucleotides were synthesized on a 1.0 µmol scale. Oligomer 32 was the parent compound having normal phosphodiester inter-nucleotide linkages. Oligomer 33 incorporated phosphorothioate inter-nucleotide linkages in the basic oligonucleotide sequence. Oligomer 34 is a an intermediate oligonucleotide having a 5'-aminolink at the 5'-terminus of the basic oligonucleotide sequence and Oligomer 35 was a similar 5'-aminolink compound incorporating phosphorothioate inter-nucleotide linkages. Oligomer 36 is a 5'-terminus cholic acid conjugate of the basic phosphodiester oligonucleotide sequence while Oligomer 37 is a similar 5'-cholic acid conjugate incorporating phosphorothioate inter-nucleotide linkages. Oligomers 32 and 33 were synthesized in a "Trityl-On" mode and were purified by HPLC. Oligomers 34 and 35 were synthesized as per Example 30-A above without or with Beaucage reagent treatment, to yield phosphodiester or phosphorothioate inter-nucleotide linkages, respectively. Oligomers 36 and 37 were prepared from samples of oligomers 34 and 35, respectively, utilizing a solution of cholic acid N-hydroxysuccinimide ester (Compound 1) 1 dissolved in DMF as per Example 3-B. Oligomers 36 and 37 were purified by HPLC. The products were concentrated and desalted in a Sephadex® G-25 column. Gel electrophoresis analyses also confirmed a pure product with the pure conjugate moving slower than the parent oligonucleotide or 5'-amino functionalized oligonucleotide.

### b. Melting Analysis

The test oligonucleotides [either the phosphodiester, phosphorothioate, cholic acid conjugated phosphodiester, cholic acid conjugated phosphorothioate or 5'-aminolink intermediate phosphodiester or phosphorothioate oligonucleotides of the invention or otherwise] and either the complementary DNA or RNA oligonucleotides were incubated at a standard concentration of 4 µM for each oligonucleotide in buffer (100 mM MaCl, 10 mM Na-phosphate, pH 7.0, 0.1 mM EDTA). Samples were heated to 90 degrees C and the initial absorbance taken using a Guilford Response II spectrophotometer (Corning). Samples were then slowly cooled to 15 degrees C and then the change in absorbance at 260 nm was monitored during the heat denaturation procedure. The temperature was elevated 1 degree/absorbance reading and the denaturation profile analyzed by taking the 1st derivative of the melting curve. Data was also analyzed using a two-state linear regression analysis to determine the Tm's. The results of these tests are shown in Table 3 as are the HPLC retention times of certain of the test compounds.

**TABLE 3**

| **Melting Temperature Of The Hybridization Complex Of The Oligonucleotide And Its Complementary Strand** | | | |
|---|---|---|---|
| **Oligomer** | **Tm**** | | **HPLC Ret. Time* minutes** |
| | **DNA** | **RNA** | |
| 32 | 62.6 | 62.0 | ---- |
| 33 | 55.4 | 54.9 | ---- |
| 34 | ND | ND | 13.6 |
| 35 | ND | ND | 17.0 |
| 36 | 63.4 | 62.4 | 22.0 |
| 37 | 56.3 | 55.8 | 22.5 |

| | | | |
|---|---|---|---|
| * HPLC conditions: Walters Delta Pak C-18 RP 2.5u column; at 0 min 100% 0.1 TEAA; at 30 min 50% TEAA and 50% Acetonitrile: Flow rate 1.0 ml/min. | | | |
| ** Tm at 4µM each strand from fit of duplicate melting curves to 2-state model with linear sloping base line. Conditions: 100 mM NaCl, 10 mM Phosphate, 0.1 mM EDTA, pH 7.0. ND = not determined | | | |

As is evident from Table 2, conjugates of cholic acid at the end of the oligonucleotide do not affect the Tm of the oligonucleotides.

### 2. Strands Incorporating 2'-O-Pentylamino Linker

### a. synthesis

An oligonucleotide of the sequence: Oligomer 38:   GGA* CCG GA*A* GGT A*CG A*G wherein A* represents a nucleotide functionalized to incorporate a pentylamino functionality at its 2'-position was synthesized in a one micromole scale utilizing the method of Example 7-B. The oligonucleotide was purified by reverse phase HPLC, detritylated and desalted on Sephadex® G-25. PAGE gel analysis showed a single band. A further oligonucleotide, Oligomer 39, having the same sequence but without any 2'-O-amino linker was synthesis in a standard manner. A complementary DNA oligonucleotide of the sequence: Oligomer 40:   CCT GGC CTT CCA TGC TC. was also synthesized in a standard manner as was a complementary RNA oligonucleotide of the sequence:

Oligomer 41:   CCU GGC CUU CCA UGC UC

### b. Melting Analysis

Melting analysis was conducted as per the method of Procedure B-i-b. The results are shown in Table 4.

**TABLE 4**

| **Melting Temperature Of The Hybridization Complex Of The Oligonucleotide And Its Complementary Strand** | | |
|---|---|---|
| Oligomer | Tm* | |
| | DNA¹ | RNA² |
| 38 | 54.5 | 58.0 |
| 39 | 60.6 | 56.9 |

| | | |
|---|---|---|
| * Tm at 4µM each strand from fit of duplicate melting curves to 2-state model with linear sloping base line. Conditions: 100 mM NaCl, 10 mM Phosphate, 0.1 mM EDTA, pH 7.0. | | |
| ¹ Against DNA complementary strand, Oligomer 40. | | |
| ² Against RNA complementary strand, Oligomer 41 | | |

As is evident from Table 4 against the RNA complementary strand the change in Tm's between the strand having 2'-amino linkers thereon and the unmodified strand is 1.1 degrees (0.22 change per modification). Against the DNA strand, the change is -6.1 degrees (-1.2 change per modification). When compared to the parent unmodified oligonucleotide the 2'-amino linker- containing strand has a stabilizing effect upon hybridization with RNA and a destabilizing effect upon hybridization with DNA.

Compounds of the invention were tested for their ability to increase cellular uptake. This was determined by judging either their ability to inhibit the expression of bovine papilloma virus-1 (BPV-1) or an assay involving luciferase production (for HIV-1).

### PROCEDURE C

### Determination of Cellular Uptake Judged By The Inhibition Of Expression of Bovine Papilloma Virus-1 (bpv-1) As Measured By an E2 Transactivation Assay

For this test, a phosphorothioate oligonucleotide analog of the sequence: Oligomer 42: CTG TCT CCA TCC TCT TCA CT was used as the basic sequence. This sequence is designed to be complementary to the translation initiation region of the E2 gene of bovine papilloma virus type 1 (BPV-1). Oligomer 42 served as the positive control and standard for the assay. Oligomer 3 (from Example 4 above) served as a second test compound. It has the same basic sequence except it is a phosphorothioate oligonucleotide and further it has a cholic acid moiety conjugated at the 3'-end of the oligonucleotide. Oligomer 2 (from Example 2 above) served as a third test compound. Again it is of the same sequence, it is a phosphorothioate oligonucleotide and it has a cholic acid moiety conjugated at the 5'-end. Oligomer 5 (from Example 5 above) served as a fourth test compound. Once again it has the same sequence, is a phosphorothioate oligonucleotide and it has a cholic acid moiety conjugated at both the 3'-end and 5'-end. A fifth test compound was a phosphorothioate oligonucleotide with no significant sequence homology with BPV-1. A sixth test compound was a further phosphorothioate oligonucleotide with no significant sequence homology with BPV-1. The last test compound, the seventh test compound, was a phosphorothioate oligonucleotide with cholic acid conjugated to the 3'-end but having no significant sequence homology with BPV-1. Compounds five, six and seven served as negative controls for the assay.

For each test I-38 cells were plated at 5x10⁴ cells per cm² in 60 mm petri dishes. Eight hours after plating, medium was aspirated and replaced with medium containing the test oligonucleotide and incubated overnight. Following incubation, medium was aspirated and replaced with fresh medium without oligonucleotide and incubated for one hour. Cells were then transfected by the CaPO₄ method with 2 µg of pE2RE-1-CAT. After a four hour incubation period cells were glycerol shocked (15% glycerol) for 1 minute followed by washing 2 times with PBS. Medium was replaced with DMEM containing oligonucleotide at the original concentration. Cells were incubated for 48 hours and harvested. Cell lysates were analyzed for chloramphenicol acetyl transferase by standard procedures. Acetylated and nonacetylated ¹⁴C-chloramphenicol were separated by thin layer chromatography and quantitated by liquid scintillation. The results are expressed as percent acetylation.

Two lots of the positive control compound were found to acetylate at a level of 29% and 30%. The negative controls, test compounds five, six and seven, were found to acetylate at 59%, 58% and 47%, respectively. The 3'-cholic acid conjugate test compound, Oligomer 3, was found to acetylate to 23%, the 5'-cholic acid conjugate test compound, Oligomer 2, was found to acetylate to 36% and the test compound conjugated at both the 3'-end and the 5'-end, Oligomer 5, was found to acetylate to 27%.

The results of this test suggests that placement of a cholic acid moiety at the 3'-terminus of an oligonucleotide increase the activity. This in turn suggests that the increased activity was the result of increased cellular membrane transport.

### PROCEDURE D

### Determination of Cellular Uptake Judged By Inhibition of pHIVluc With Cholic Acid Linked 2'-O-Methyl Substituted Oligonucleotides

For this test the absence of an oligonucleotide in a test well served as the control. All oligonucleotides were tested as 2'-O-methyl analogs. For this test an oligonucleotide of the sequence: Oligomer 43:   CCC AGG CUC AGA where each of the nucleotides of the oligonucleotide includes a 2'-O-methyl substituent group served as the basic test compound. The second test compound of the sequence: Oligomer 44: 5'-CHA CCC AGG CUC AGA wherein CHA represents cholic acid and where each of the nucleotides of the oligonucleotide includes a 2'-O-methyl substituent group, was also of the same sequence as the first test compound. This second test compound included cholic acid conjugated to its 5'-end and was prepared as per the method of Example 3 utilizing 2'-O-methyl phosphoramidite intermediates as identified in Example 7-C. The third test compound of the sequence: Oligomer 45: CCC AGG CUC AGA 3'-CHA wherein CHA represents cholic acid and where each of the nucleotides of the oligonucleotide includes a 2'-O-methyl substituent group was also of the same sequence as the first test compound. The third test compound included cholic acid conjugated to its 3'-end and was prepared as per the method of Example 4 utilizing 2'-O-methyl phosphoramidite intermediates as identified in Example 7-C. The fourth test compound was a 2'-O-Me oligonucleotide of a second sequence: Oligomer 46:   GAG CUC CCA GGC where each of the nucleotides of the oligonucleotide includes a 2'-0-methyl substituent group. The fifth test compound was of sequence: Oligomer 47: 5′-CHA GAG CUC CCA GGC. wherein CHA represents cholic acid and where each of the nucleotides of the oligonucleotide includes a 2'-O-methyl substituent group. It was of the same sequence as the fifth test compound. This test compound included cholic acid conjugated to its 5'-end and was prepared as per the method of Example 3 utilizing 2'-O-methyl phosphoramidite intermediates as identified in Example 7-C.
A sixth test compound was a randomized oligonucleotide of the sequence:

Oligomer 48: CAU GCU GCA GCC.

HeLa cells were seeded at 4x10⁵ cells per well in 6-well culture dishes. Test oligonucleotides were added to triplicate wells at 1 µM and allowed to incubate at 37°C for 20 hours. Medium and oligonucleotide were then removed, cells washed with PBS and the cells were CaPO₄ transfected. Briefly, 5µg of pHIVluc, a plasmid expressing the luciferase cDNA under the transcriptional control of the HIV LTR constructed by ligating the KpnI/HindIII restriction fragments of the plasmids pT3/T71uc and pHIVpap (NAR 19(12)) containing the luciferase cDNA and the HIV LTR respectively, and 6 µg of pcDEBtat, a plasmid expressing the HIV tat protein under the control of the SV40 promoter, were added to 500 µl of 250 mM CaCl₂, then 500 µl of 2x HBS was added followed by vortexing. After 30 minutes, the CaPO₄ precipitate was divided evenly between the six wells of the plate, which was then incubated for 4 hours. The media and precipitate were then removed, the cells washed with PBS, and fresh oligonucleotide and media were added. Incubation was continued overnight. Luciferase activity was determined for each well the following morning. Media was removed, then the cells washed 2X with PBS. The cells were then lysed on the plate with 200 µl of LB (1% Trit X-100, 25 mM Glycylglycine pH 7.8, 15 mM MgSO₄, 4 mM EGTA, 1mM DTT). A 75 µl aliquot from each well was then added to a well of a 96 well plate along with 75 µl of assay buffer (25 mM Glycylglycine pH 7.8, 15 mM MgSO₄, 4 mM EGTA, 15 mM KPO₄, 1 mM DTT, 2.5 mM ATP). The plate was then read in a Dynatec multiwell luminometer that injected 75 µl of Luciferin buffer (25 mM Glycylglycine pH 7.8, 15 mM HgSO₄, 4 mM EGTA, 4 mM DTT, 1 mM luciferin) into each well, immediately reading the light emitted (light units).

The random sequence compound (Oligomer 48) and the other non-cholic acid-conjugated test compounds (Oligomers 43 and 46) had comparable activity. The 5'-conjugate of the first sequence (Oligomer 44) also had activity comparable to the non-conjugated compounds. The 5'-conjugate of the second sequence (Oligomer 47) showed a three-fold increase in activity compared to the non-conjugated compounds and the 3'-conjugate of the first sequence (Oligomer 45) showed a further 3-fold increase in activity compared to Oligomer 47.

All the test cholic acid-bearing oligonucleotides showed significant inhibition of luciferase production compared to non-cholic acid- bearing oligonucleotides. This suggests that the increased activity was the result of increased cellular membrane transport of the cholic acid-bearing test oligonucleotides.

### EXAMPLE 13

### Retinoic Acid conjugated oligonucleotide

### A. Retinoic Acid N-Hydroxysuccinimide Ester

Anhydrous DMF (150 ml) was added to a mixture of retinoic acid (15 mmol, 4.5 g, Fluka) and N-hydroxysuccinimide (5.25 g, 45 mmol). The mixture was stirred in the presence of argon. EDAC [ethyl-3-(3-dimethylamino)propyl carbodiimide] (4 ml, 25 nmol) was then added and this mixture was then stirred overnight. The solution was then evaporated to a yellow gum and dissolved in 200 ml ethylacetate and washed successively with 4% NaHCO₃ solution (200 ml) followed by saturated NaCl solution, dried over anhydrous MgSO₄ and evaporated to yield the desired compound as a yellow solid in nearly 90% yield.

### B. Retinol Phosphoramidite

All-trans-retinol (1 g) was vacuum dried and dissolved in anhydrous CH₂Cl₂ (10 ml) in an argon atmosphere. Diisopropylethylamine (2.65 ml, 21.5 mmol) was syringed in and the reaction mixture was cooled in an ice-bath. 2-cyanoethyl-N, N-diisopropylchlorophosphoramidate (2.5 g, 2.45 ml, 10.5 mmol) was slowly added by syringe under argon atmosphere. The reaction mixture was stirred for 30 min. at which time TLC (CH₂Cl₂:CH₃OH:Et₃N, 90:10:0.1) indicated complete conversion of the alcohol to its phosphoramidite. The reaction mixture was added to 100 ml of saturated NaHCO₃ followed by washing the reaction flask with (2 x 25 ml) CH₂Cl₂. The CH₂Cl₂ layer was separated and washed with 100 ml of saturated NaCl solution and dried over anhydrous MgSO₄ and evaporated into a yellow foam. The amidite was used directly in the DNA synthesizer without further purification since decomposition of this amidite was noted upon silica column purification.

### C. Retinoic Acid Functionalized Oligonucleotide

Multiple batches of an oligonucleotide of the sequence: 
Oligomer 49:
TsGsGs GsAsGs CsCsGs TsAsGs CsGsAs GsGsCs-3'AL
 wherein AL represents a 3'-aminolinker and "s" represents a phosphorothioate inter-nucleotide backbone linkage were synthesized as per the procedure of Example 4 on 10 µmol scales in the standard manner on the DNA synthesizer utilizing phosphoramidite methodology employing 3'-amine-ON solid support available from Clontech. During the synthesis, the phosphorothioate backbone was formed by the Beaucage reagent. The oligonucleotide was deprotected and purified using standard protocols.

The 3'-aminolinker-oligonucleotide (Oligomer 49, 100 OD units, approximately 550 nmols) was dissolved in freshly prepared NaHCO3 buffer (500 µl, 0.2M, pH 8.1) and treated with a solution of retinoic acid N-hydroxy succinimide ester (50 mg, 125 µmols) dissolved in 500 µl of DMF. The reaction mixture was covered with aluminum foil and left at 37°C bath overnight. It was then passed through a Sephadex® G-25 column (1 x 40 cm) and the first eluant was collected, concentrated and passed again through another Sephadex® G-25 column (1 x 40 cm) to remove excess Vitamin-A reagent. Concentration of the yellow oligonucleotide fractions followed by HPLC purification yielded the retinoic acid-oligonucleotide conjugate.

### EXAMPLE 14

### Folic Acid Conjugated Oligonucleotide

A mixture of folic acid (30 mg, 68 µmols) and 1-hydroxybenzotriazole (30 mg, 222 µmols) was dissolved in 900 ml of dry DMF. To this solution, 50 ml of EDAC (312 µmols) was added. The resultant yellow viscous material was vortexed well and 500 ml from the solution was transferred into 100 O.D. units of the 3'-aminolinker oligonucleotide (Oligomer 49, 537 µmols) dissolved in 0.2M NaHCO₃ buffer. The yellow solution was vortexed, covered with aluminum foil and allowed to react for 16 hrs. The mixture was then loaded into a Sephadex® G-25 column (1 x 40 cm). The oligonucleotide fraction was collected, concentrated and passed one more time through Sephadex® G-25 column. The oligonucleotide fractions were concentrated and purified by reverse phase HPLC. The conjugate appeared as a broad peak centered around 32.5 min. while the oligonucleotide starting material had a retention time of 30 min. (5% → 40% of 100% CH₃CN over 60 min. as solvent B and 50 mM TEAA pH 7.0 as the solvent A in reverse phase HPLC). The conjugate was concentrated and passed through Sephadex® G-25 column again for desalting. Gel analysis indicated a slower moving material than the starting oligonucleotide.

### EXAMPLE 15

### Methyl Folate Conjugated Oligonucleotide

In a like manner to Example 14, 5-methyl folate was also attached to Oligomer 49.

### EXAMPLE 16

### Pyridoxal Conjugated Oligonucleotide

The 3'-aminolinker-oligonucleotide (Oligomer 49, 20 O.D. units, approximately 110 nmols, based on the calculated extinction coefficient of 1.828 x 10⁵ at 260 nm) was dissolved in 100 microliters of water. 100 ml of 1M NaOAc buffer (pH 5.0) was added followed by 5 mg of pyridoxal hydrochloride (24 µmols) and 50 µl of 60 mM NaCNBH3 solution. The solution was vortexed and left aside for overnight. It was then passed through a Sephadex® G-25 column and further purified in an analytical HPLC column.

### EXAMPLE 17

### Tocopherol Conjugated Oligonucleotide

### A. vitamin E (Tocopherol)-hemisuccinate-NHS ester

α-Tocopherolhemisuccinate (Sigma, 5 g, 9.4 mmols) was treated with 3 equivalents of N-hydroxysuccinimide and 2 equivalents of EDAC as described under the vitamin-A NHS ester synthesis, Example 13-A above. Work up in the same manner as Example 13 yielded the title compound as a light brown wax-like solid.

### B. Tocopherol Conjugated oligonucleotide

α-Tocopherol-hemisuccinate-NHS ester was treated with Oligomer 49 in the same manner described in Example 13-C for the retinoic acid conjugation. The conjugate was obtained in nearly 50% yield.

### EXAMPLE 18

### Synthesis of Uridine Based Aminolinkers

### A. Preparation of 5'-dimethoxytrityl-2'-(O-pentyl-N-phthalimido)uridine phosphoramidite

Utilizing the protocol of Wagner, *et al., J. Org. Chem.* **1974,** *39*, 24, uridine (45 g, 0.184 mol) was refluxed with di-n-butyltinoxide (45 g, 0.181 mol) in 1.4 1 of anhydrous methanol for 4 hrs. The solvent was filtered and the resultant 2',3'-O-dibutylstannylene-uridine was dried under vacuum at 100°C for 4 hrs to yield 81 g (93%).

The 2',3'-O-dibutyl stannylene-uridine was dried over P₂O₅ under vacuum for 12 hrs. To a solution of this compound (20 g, 42.1 mmols) in 500 ml of anhydrous DMF were added 25 g (84.2 nmols) of N(5-bromopentyl)phthalimide (Trans World Chemicals, Rockville, Maryland) and 12.75 g (85 mmols) of cesium fluoride (CeF) and the mixture was stirred at room temperature for 72 hrs. The reaction mixture evaporated, coevaporated once with toluene and the white residue was partitioned between EtOAc and water (400 ml each). The EtOAC layer was concentrated and applied to a silica column (700 g). Elution with CH₂Cl₂-CH₃OH (20:1 v/v) gave fractions containing a mixture of the 2'- and 3'- isomers of O-pentyl-ω-N-phthalimido uridine, in 50% yield.

The mixture was allowed to react with DMT chloride in dry pyridine at room temperature for 6 hrs. CH₃OH was used to quench excess DMT-Cl and the residue was partitioned between CH₂Cl₂ containing 0.5% Et₃N and water. The organic layer was dried (MgSO₄) and the residue was applied to a silica column. Elution with CH₂Cl₂:CH₃OH (20:1, v/v) separated the 2' and 3' isomers.

The 2'-O-pentyl-ω-N-phthalimido-5'-DMT-uridine was converted to its phosphoramidite as per the procedure referenced in Example 7.

### B. Preparation of 5'-dimethoxytrityl-2-(O-hexyl-N-phthalimido)uridine phosphoramidite

In a like manner to Example 18-A, using N-(6-bromohexyl) phthalimide, a 2'- six carbon aminolinker was introduced at the 2'-position of uridine.

### C. Preparation of 5'-dimethoxytrityl-2-(O-decyl-N-phthalimido)uridine pbosphoramidite

In a like manner to Example 18-A N-(10-bromodecyl)phthalimide was similarly used to introduce a 2'-ten carbon aminolinker in the nucleotide.

### EXAMPLE 19

### Synthesis of Cytidine Based Aminolinkers

### A. Preparation of 5'-dimethoxytrityl-2-(O-propyl-N-phthalimido)cytidine phosphoramidite

The 5'-DMT protected 2'-O-functionalized cytidine phosphoramidite was prepared as per the procedure of Example 7 substituting cytidine for adenosine.

### B. Preparation of oligonucleotides having a 2'-aminolinker bearing 3'-terminal nucleotide

The following oligonucleotides having phosphodiester inter-nucleotide linkages and a 2'-aminolinker at the 3' terminal nucleotide were synthesized: Oligomer 50:   GGC GUC UCC AGG GGA UCU GAC* Oligomer 51: TCT GAG TAG CAG AGG AGC TC* wherein C* represents a nucleotide functionalized to incorporate a propyl-N-phthalimido functionality. Oligomer 50 is antisense to the Cap region of CMV and Oligomer 51 is antisense to an ICAM sequence. The oligonucleotides were synthesized on a 3 µmol scale. Upon completion of synthesis they were deprotected using standard protocols and purified by reverse phase HPLC, detritylated and desalted.

### EXAMPLE 20

### Conversion Of An Oligonucleotide Having A 2'-Aminolinker To An Oligonucleotide Having A Thiolinker

### A. Oligomer 50

Oligomer 50 (25 O.D. units) was treated with 5 mg SATA (N-succinimidyl-S-acetylthioacetate) in 0.2M NaHCO₃ buffer. The reaction mixture was passed through a Sephadex® G-25 column, the oligonucleotide fraction was concentrated and treated with 200 mM NH₂OH hydrochloride solution in water (1 ml).

### B. Oligomer 51

Oligomer 51 (25 O.D. units) was treated with 5 mg SATA (N-succinimidyl-S-acetylthioacetate) in 0.2M NaHCO₃ buffer. The reaction mixture was passed through a Sephadex® G-25 column, the oligonucleotide fraction was concentrated and treated with 200 mM hydroxylamine hydrochloride solution in water (1 ml).

### EXAMPLE 21

### Conjugation of o-Phenanthroline at 2'-Position of Oligonucleotides

### A. Oligomer 52

To the solution resulting from Example 20-A was added 2 mg of 5-(iodoacetamide)-o-phenanthroline reagent followed by shaking overnight. The conjugate was purified by a size exclusion column and reverse phase HPLC to yield Oligomer 52:   GGC GUC UCC AGG GGA UCU GAC-2'PHA wherein PHA represents a nucleotide functionalized at its 2'-position with phenanthroline via a thiol linker of the structure 2'-O-(CH₂)₃-NH-C(=O)-CH₂-S-CH₂-C(=O)-NH-.

### B. Oligomer 53

To the solution resulting from Example 20-A was added 2 mg of 5-(iodoacetamide)-O-phenanthroline reagent followed by shaking overnight. The conjugate was purified by a size exclusion column and reverse phase HPLC to yield Oligomer 53:   TCT GAG TAG CAG AGG AGC TC-2'PHA wherein PHA represents a nucleotide functionalized at its 2'-position with phenanthroline via a thiol linker of the structure 2'-O-(CH₂)₃-NH-C(=O)-CH₂-S-CH₂-C(=O)-NH-.

### EXAMPLE 22

### Oligonucleotide Having A Nucleotide with A Crosslinker/Alkylator Attached Via A 2'-Aminolinker In A Internal Position In The Oligonucleotide

### A. Synthesis of an Oligonucleotide having a uridine 2'-aminolinker

The following oligonucleotide having phosphodiester inter-nucleotide linkages and a 2'-aminolinker at an internal position is synthesized utilizing the uridine 2'-aminolinker of Example 18: 
Oligomer 54:   GGC CAG AUC UGA GCC UGG GAG CU C UGU GGC
C
 wherein U* represents a nucleotide functionalized to incorporate a propyl-N-phthalimido functionality. Oligomer 50 is an oligonucleotide corresponding to positions G₁₆ to C₄₆ of TAR RNA.

### B. Conjugation of iodo acetamide to U₃₈ position of TAR structure

Oligomer 54 is reacted with iodoacetic acid N-hydroxysuccinimide ester to form the iodoacetamide derivative at the U₃₈ position of the TAR structure. The U₃₈ position is thus available for crosslinking to the 7 position of the guanine base of either G₂₆ or G₂₈ of the TAR structure.

### EXAMPLE 23

### Conjugation of Pyrene at 2'-Position Of Oligonucleotides

### A. Single 2' site modification

10 O.D. units (A₂₆₀) of Oligomer 12 (Example 7-B) (approximately 60 nmols based on the calculated extinction coefficient of 1.68 x 10⁵) was dried in a microfuge tube. It was dissolved in 200 µml of 0.2 M NaHCO₃ buffer and pyrene-1-butyric acid N-hydroxysuccinimide ester (*i.e*., succinimidyl-1-pyrene butyrate, 3 mg, 7.79 µmols, Molecular Probes, Eugene, Oregon) was added followed by 400 µl of DMF. The mixture was incubated at 37°C overnight. The solution was applied to a Sephadex® G-25 column (1 x 40 cm) and the oligonucleotide fractions were combined. The product was purified by HPLC. The pyrene conjugates exhibited the typical pyrene absorption between 300 and 400 nm. The product had a HPLC retention time of 26.94 min. while the parent oligonucleotide had a retention time of 21.78 min. (Waters 600E with 991 detector; Hamilton PRP-1 column (15x25 cm); Solvent A: 50 mM TEAA, pH 7.0; B: 45 mM TEAA with 80% Acetonitrile; 1.5 ml/min. flow rate: Gradient 5% B for first 5 minutes, linear (1%) increase in B every minute afterwards).

### B. Multiple 2' site modifications

10 O.D. units of Oligomer 12 (Example 7-B) was treated with twice the amount of pyrene-1-butyric acid N-hydroxysuccinimide (6 mg in 400 µl DMF) and worked up in the same fashion as Example 23-A. Sephadex® G-25 purification followed by HPLC purification gave the doubly pyrene-conjugated oligonucleotide. The doubly conjugated oligonucleotide exhibited a HPLC retention time of 32.32 min. while the parent oligonucleotide had a retention time of 21.78 min. (Waters 600E with 991 detector; Hamilton PRP-1 column (15x25 cm); Solvent A: 50 mM TEAA, pH 7.0; B: 45 mM TEAA with 80% Acetonitrile; 1.5 ml/min. flow rate: Gradient 5% B for first 5 minutes, linear (1%) increase in B every minute afterwards).

### EXAMPLE 24

### Conjugation of Acridine at 2'-Position Of Oligonucleotides

### A. Single 2' site modification

10 O.D. units (A₂₆₀) of Oligomer 12 (Example 7-B, about 60 nmols) was dried and dissolved in 1M NaHCO₃/Na2CO₃ buffer, pH 9.0, 200 µl. 9-acridinyl-isothiocyante, (5 mg, 2.1 µmols, Molecular Probes, Eugene, Oregon) was dissolved in 200 µl DMF. This solution was added to the oligonucleotide, vortexed, covered with aluminum foil and left at 37°C overnight. The reaction mixture was purified by passing through a Sephadex® G-25 column (1 x 40 cm) concentrated and further purified by HPLC (reverse-phase). The product had a HPLC retention time of 25.32 min. while the parent oligonucleotide had a retention time of 21.78 min. (Waters 600E with 991 detector; Hamilton PRP-1 column (15x25 cm); Solvent A: 50 mM TEAA, pH 7.0; B: 45 mM TEAA with 80% Acetonitrile; 1.5 ml/min. flow rate: Gradient 5% B for first 5 minutes, linear (1%) increase in B every minute afterwards).

### B. Multiple 2' site modifications

10 O.D. units (A₂₆₀) of Oligomer 13 (Example 7-B) in 400 µl of 1M Na2CO₃/NaHCO₃ buffer (pH 9.0) was treated with 10 mg of 9-acridinyl-isothiocyanate in 400 µl of DMF. The reaction mixture was vortexed, covered with aluminum foil and left at 37°C overnight. The reaction mixture was purified as for the single site reaction of Example 24-A. The doubly conjugated acridine-oligonucleotide eluted as the last peak in the HPLC following single-modification products. The product had a HPLC retention time of 32.32 min. while the parent oligonucleotide had a retention time of 21.78 min. (Waters 600E with 991 detector; Hamilton PRP-1 column (15x25 cm); Solvent A: 50 mM TEAA, pH 7.0; B: 45 mM TEAA with 80% Acetonitrile; 1.5 ml/min. flow rate: Gradient 5% B for first 5 minutes, linear (1%) increase in B every minute afterwards).

### EXAMPLE 25

### Conjugation Of Porphyrin at 2'-Position Of Oligonucleotides

Methylpyroporphyrin XX1 ethyl ester (Aldrich) is condensed with aminocaproic acid using N-hydroxysuccinimide and EDAC. The resultant carboxylic acid is then activated again with N-hydroxy succinimide and EDAC and treated with Oligomer 12 as per the procedure of Example 23-A to give the 2'-porphyrin conjugated oligonucleotide.

### EXAMPLE 26

### Conjugation of Hybrid Intercalator-Ligand at 2'-Position Of oligonucleotides

### A. Photonuclease/intercalator ligand

The photonuclease/intercalator ligand 6-[[[9-[[6-(4-nitrobenzamido)hexyl]amino]acridin-4-yl]carbonyl]amino]hexanoyl-pentafluorophenyl ester was synthesized as per the procedure of Egholm *et al., J. Am. Chem. Soc.* **1992,** *114*, 1895.

### B. Single 2' site modification

10 O.D. units of Oligomer 12 (Example 7-B) was dissolved in 100 µl of 0.1 M borate buffer (pH 8.4) and treated with 330 µl of DMF solution (10 mg in 1 ml of DMF) of 6-[[[9-[[6-(4-nitrobenzamido)hexyl]amino]acridin-4-yl]carbonyl]amino]hexanoylpentafluorophenyl ester. The solution was covered with aluminum foil and allowed to react overnight. The product was purified by Sephadex® G-25 and HPLC purification of the reaction mixture.

### C. Multiple 2' site modification

10 O.D. units A₂₆₀ of Oligomer 13 (Example 7-B) was dissolved in 200 µl of 0.1 M borate buffer (pH 8.4) and treated with 660 µl of the DMF solution of 6-[[[9-[[6-(4-nitrobenzamido)hexyl]amino]acridin-4-yl]carbonyl]amino]hexanoylpentafluorophenyl ester (10 mg in 1 ml solution) and the solution was covered with aluminum foil and left aside overnight. The bright yellow solution was purified by Sephadex® G-25 and reverse phase HPLC to give the doubly conjugated oligonucleotide.

### EXAMPLE 27

### Conjugation of Bipyridine Complex at 2'-Position Of Oligonucleotides

### A. Bipyridine complex

Succinimidyl-4-carboxyl-4'-methyl-2,2'-bipyridine is synthesized according to the procedure of Telser, *et al., J. Am. Chem. Soc.* **1989,** *111*, 7221.

### B. Single 2' site Modification

10 O.D. A₂₆₀ units of Oligomer 12 is reacted with a 200 fold molar excess of succinimidyl-4-carboxyl-4'-methyl-2,2'-bipyridine in 0.1 M borate buffer pH 8.5/DMF. The solution is purified by Sephadex® G-25 and reverse phase HPLC to yield the conjugated oligonucleotide.

### EXAMPLE 28

### Conjugation of Aryl Azide Photocrosslinkers at 2'-Position Of Oligonucleotides

### A. Conjugation of N-hydroxysuccinimidyl-4-azidobenzoate (HSAB)

Oligomer 14 (*i.e*., TTG CTT CCA* TCT TCC TCG TC wherein A* represents 2'-O-pentyl amino adenosine, Example 7-C, 100 O.D. units, 595 nmols, based on the calculated extinction coefficient of 1.6792 x 10⁶ OD units) was dried and dissolved in 500 ml of 0.2M NaHCO₃ buffer pH 8.1 and treated with 25 mg of N-hydroxysuccinimidyl-4-azidobenzoate (HSAB, 96 µmols, available both from Pierce & Sigma)) dissolved in 500 µl of DMF. The reaction was allowed to react overnight at 37°C and passed twice over Sephadex® G-25 column (1 x 40 cm). The oligonucleotide fraction was purified by reverse-phase HPLC. The product had the HPLC retention time of 38.79 min while the parent oligonucleotide had the retention time of 33.69 min. (5% → 40% CH3CN in 60 min.) in reverse phase column.

### B. Conjugation of N-succinimidyl-6(4'-azido-2'-nitrophenyl-amino)hexanoate

Oligomer 14 (Example 7-C, 200 OD units) was dissolved in 500 ml NaHCO₃ buffer (0.2M, pH 8.1) and treated with 500 mg of N-succinimidyl-6(4'-azido-2'-nitrophenyl-amino)hexanoate (SANPAH, 128 µmols, available both from Pierce and Sigma) dissolved in 500 µl DMF. The reaction vial was wrapped with aluminum foil and heated at 37°C overnight. The reaction mixture was passed twice over a Sephadex® G-25 column (1 x 40 cm). The oligonucleotide fraction was purified by reverse-phase HPLC. The product had the HPLC retention time of 40.69 min. while the parent oligonucleotide had the retention time of 33.69 min. (5% → 40% CH₃CN in 60 min.) in a reverse phase column.

### PROCEDURE D

### Duplex Melting Temperature of conjugated Oligonucleotides

Utilizing the protocol described in Procedure B-1-b, the melting temperatures of various of the 2-aminolinked conjugate oligomers of the invention against a complementary DNA strand were obtained. The conjugate oligomers were compared to an oligomer of the same sequence bearing a 2'-O-pentylamino group. An un-modified, *i.e*., wild type, strand of the same sequence was also tested for comparison purposes. Both single site and multiple site conjugated oligomers were tested. As is shown in Table 5, the Tₘ and the ΔTₘ/modification, both as compared to 2'-pentylamino bearing oligomer, were measured. The wild type sequence is: 
Oligomer 55:
CTG TCT CCA TCC TCT TCA CT
 the single site sequence is: 
Oligomer 12:
CTG TCT CCA* TCC TCT TCA CT
 and the multiple site sequence is: 
Oligomer 13:
CTG TCT CCA* TCC TCT TCA* CT
 where A* represents a site of conjugation.

**TABLE 5**

| **Duplex Melting Temperature of Conjugated Oligonucleotides Against DNA** | | | |
|---|---|---|---|
| Oligomer | Conjugate | Tₘ°C | ΔTₘ/mod |
| 55 | wild type | 60.5 | -- |
| 12 | 2'-O-pentyl-NH₂ | 58.1 | -- |
| 12 | biotin | 56.4 | -1.7 |
| 12 | cholic acid | 55.5 | -2.6 |
| 12 | digoxigenin | 55.8 | -2.3 |
| 12 | fluorescein | 55.1 | -3.0 |
| 12 | pyrene | 62.6 | +4.5 |
| 12 | acridine | 58.6 | +0.5 |
| 13 | 2'-O-pentyl-NH₂ | 56.9 | -- |
| 13 | biotin | 54.4 | -1.3 |
| 13 | cholic acid | 54.3 | -1.3 |
| 13 | digoxigenin | 53.8 | -1.6 |
| 13 | fluorescein | 53.4 | -1.8 |
| 13 | pyrene | 65.1 | +4.1 |
| 13 | acridine | 58.1 | +1.2 |

### EXAMPLE 29

### Conjugation of Imidazole-4-acetic acid at 2'-Position of Oligonucleotides

### A. Activated imidazole-4-acetic acid

Imidazole-4-acetic acid was reacted with 2,4-dinitrofluoro benzene. The resulting imidazole-N-(DNP)-4-acetic acid was converted to its N-hydroxy succinimide ester by treating with NHS/EDAC as per the procedure of Example 13.

### B. 2'-Site Modification

10 O.D. A₂₆₀ units of oligomer 12 was reacted with a 100 fold molar excess of imidazole-N-(DNP)-4-acetic acid NHS ester in 0.1M borate buffer pH 8.5/DMF (200 µl each). After overnight reaction, the reaction mixture was treated with 200 µl of mercaptoethanol to cleave off the DNP protecting group. The resulting reaction mixture was purified by passing through a Sephadex® G-25 column followed by reverse phase HPLC to yield the imidazole conjugated oligonucleotide.

### EXAMPLE 30

### Conjugation of Metal Chelating Agents to 2'-Position of Oligonucleotide

### A. EDTA Complex

To form an EDTA Fe(II) complex for coupling to an oligonucleotide as a nucleic acid cleaving agent, tricylohexyl ester of EDTA is synthesized according to the procedure of Sluka, *et al., J. Am. Chem. Soc.* **1990,** *112*, 6369.

### B. EDTA 2'-Site Modification

The tricyclohexyl ester of EDTA (1.25 mg, 1.94 mmol) and hydroxybenzotriazole (HOBt, 1 mg, 6.6 mmol) are dissolved in DMF (50 µl) and EDAC 10 µl is added. To this solution, oligonucleotide 12 (10 OD units) in 100 µl g 0.1M borate buffer is added and left overnight. The solution is passed through a Sephadex® G-25 column and the oligonucleotide fraction treated with conc. NH₃ (100 µl) for 1 hr. to cleave off the acid protecting groups. Finally purification is effected by size exclusion and HPLC.

### C. DTPA 2'-Site Modification

Oligomer 12 was treated with diethylene triamine pentaacetic anhydride (DTPA) in 0.1M NaHCO₃/DMF to offer single-site modification. The conjugate was complexed with Gadolinium ion (Gd III) to give a contrast agent, usable among other uses, as an uptake measuring agent.

### EXAMPLE 31

### Conjugation of Cholesterol To The 2'-Position of Oligonucleotide

### A. Method 1 - 2'-Aminolinker

Cholesterol-hemisuccinate was converted to its N-hydroxy succinimide ester. It was then conjugated to Oligomer 12 as per the procedure of Example 23-A or Oligomer 13 as per the procedure of Example 23-B.

### B. Method 2 - Conjugation of Cholesterol via a Disulfide Bridge

### Step 1

Thiocholesterol (1.4 g,3.5 mmol) is added to a stirred solution of 2,2'-dithiobis(5-nitropyridine) (1.4 g 4 mmol) in chloroform 20 ml containing glacial acetic acid (400 µl) under an argon atmosphere. The reaction is allowed to continue overnight at room temperature, after which the precipitated 5-nitropyridine-2-thione was removed and the solution evaporated and purified in a silica column to give S-(2-thio-5-nitropyridyl)-thio cholesterol.

### Step 2

Oligomer 55:
TsGsGs GsAsGs CsCsGs TsA*sGs CsGsAs GsGsCs

wherein A* represents an adenosine nucleotide funtionalized to incorporate a 2'-O-pentylamino linking group is synthesized as per Example 7. This oligonucleotide is then converted into a thiol linker compound as per the procedure described for Oligomer 50 in Example 20.

The thiol linker group containing oligonucleotide, Oligomer 55, is reacted with an excess of S-(2-thio-5-nitropyridyl)-thiocholesterol to conjugate the cholesterol moiety to the 2' position of the oligonucleotide via a disulfide bridge.

### EXAMPLE 32

### Synthesis of 2'-Aminolinker containing Solid Supports for DNA/RNA synthesis

### A. 5'-Dimethyoxytrityl-2'-O-(pentyl-N-phthalimido)uridine

5'-Dimethyoxytrityl-2'-O-(pentyl-N-phthalimido)uridine was synthesized as per the procedure described in Example 18.

### B. Succinate Nucleoside

The nucleoside of step A (1 mmol) was treated with 4-DMAP (122 mg, 1 mmol) and succinic anhydride (250 mg, 2.5 mmol) in 10 ml g anhydrous pyridine. After shaking overnight, TLC (EtOAc: Hexane 6:4 with 0.1% Et₃N) indicated complete succinylation of the nucleoside. 10 ml of water was added and the reaction shaken for an additional 1 hr. The reaction mixture was evaporated and partitioned between CHCl₃ and 20% citric acid (50 ml each). The chloroform layer was washed with brine and evaporated. It was then used in the next step.

### C. Nitrophenyl Succinate

The dry 3'-O-succinate from step B was dissolved in dry dioxan (10 ml) containing pyridine (400 ml). 4-Nitrophenol (280 mg, 2 mmol) was added followed by DCC (1.32 g, 5 mmol) and the solution was shaken for 24 hrs. The fine precipitate of urea was filtered and the filtrate evaporated and applied to a silica column. The column was eluted with 5% CH₃OH in CHCl₃ containing 0.1% Et₃N. The 3'-nitrophenyl succinate of the nucleoside eluted first from the column. The product containing fractions were evaporated to give a foam.

### D. Nucleoside Solid Support

The 3'-nitrophenyl succinate from step C was dissolved in 5 ml of anhydrous DMF and treated with 5g of 500 ^{°}A pore diameter aminopropyl CPG support and shaken for 8 hrs. The CPG support was filtered, washed with methanol (5 x 20 ml) followed by ether (5 x 20) and dried. The CPG support was capped by treating for 30 min. with pyridine/acetic anhydride/N-methyl imidazole (20 ml, 8:1:1 v/v/v). The CPG support was then filtered off, washed with pyridine, methanol and ether and air-dried. Assay of the dimethoxy trityl showed the loading capacity of the CPG support was 27 mmols/gram.

### PROCEDURE E

### Determination of Cellular Uptake of Folic Acid Conjugated Oligonucleotide

The effect of conjugation of an oligonucleotide with folic acid was determined by the inhibition of ICAM-1 utilizing the method of Chiang, *et al., J. Biol. Chem.* **1991**, *266*, 18162. Utilizing this method, human lung epithelial carcinoma cells (A549 cells) were grown to confluence in 96 well plates. Medium was removed and the cells were washed with folic acid free medium three times. Folic acid free medium was added to the cells and increasing concentrations of an ICAM-1 specific antisense phosphorothioate oligonucleotide having the sequence 5'-TGG GAG CCA TAG CGA GGC-3', either free or conjugated to folic acid, was added to the incubation medium. This oligonucleotide is an 18 base phosphorothioate oligonucleotide that targets the AUG translation initiation codon of the human ICAM-1 mRNA (Chiang *et al., J. Biol. Chem.* **1991,** *266*, 18162). The oligonucleotides were incubated with the A549 cells for 24 hours then ICAM-1 was induced by adding 2.5 ng/ml tumor necrosis factor-a to the medium. Cells were incubated an additional 15 hours in the presence of tumor necrosis factor-α and oligonucleotide. ICAM-1 expression was determined by a specific ELISA as described by Chiang, *et al*.. We had previously demonstrated that the addition of the test oligonucleotide to incubation medium alone does not result in inhibition of ICAM-1 expression. However formulation of the test oligonucleotide with cationic liposomes results in at least a 1000 fold increase in potency and also correlates with the appearance of the oligonucleotide in the nucleus (Bennett, *et al., Molecular Pharmacology* **1991,** 41, 1023). The results of this test are shown in Table 6. At the 30uM level, the folic acid conjugated oligonucleotide shows an approximate 40% enhancement in activity.

**TABLE 6**

| **ICAM-1 Activity** | | |
|---|---|---|
| **Concentration** | **Oligonucleotide Control** | **Oligonucleotide + Folic Acid - Percent Of Control** |
| 1 uM | 105.1 | 107.6 |
| 3 uM | 112.0 | 104.4 |
| 10 uM | 112.4 | 92.9 |
| 30 uM | 108.4 | 61.6 |

## Claims

1. A compound comprising a plurality of linked nucleosides, wherein:
at least one of said nucleosides is functionalized at its 2'-position by direct attachment of a substituent selected from the group consisting of a steroid molecule, a reporter enzyme, a peptide, a protein, a pyrene, a water soluble vitamin, a lipid soluble vitamin, a metal chelator, a porphyrin, an alkylator, a hybrid photonuclease/intercalator, and an aryl azide photo-crosslinking agent, or
at least one of said nucleosides is functionalized at its 2'-position by attachment, through a linking moiety, of a substituent selected from the group consisting of a reporter enzyme, a peptide, a protein, a water soluble vitamin, a lipid soluble vitamin, an RNA cleaving complex, a metal chelator, an alkylator, and a hybrid photonuclease/intercalator, provided that said linking moiety does not include a disulfide group.

2. The compound of claim 1, wherein said functionalized nucleoside comprises a steroid molecule, biotin, a reporter enzyme, a peptide, a protein, a pyrene, thiamine, riboflavin, nicotinic acid, niacin, pyridoxal, panthothenic acid, biotin, folic acid, methyl folate, a vitamin B₁₂ cobamide coenzyme, inositol, choline, ascorbic acid, retinoic acid, retinol, a tocopherol, vitamin D, vitamin K, EDTA, DTPA, o-phenanthroline, a photonuclease/intercalator, an aryl azide photo-crosslinker, a phenanthroline/metal complex or a bipyridine/metal complex attached to the 2'-position of said nucleoside.

3. The compound of claim 1 or claim 2, wherein said linking moiety comprises an Ω-aminoalkoxy or Ω-aminoalkylamino moiety.

4. The compound of claim 1, wherein cholic acid, deoxycholic acid, dehydrocholic acid, cortisone, testosterone, cholesterol or digoxigenin is attached at the 2'-position of said nucleoside.

5. A compound comprising a plurality of linked nucleosides, wherein at least one of the nucleosides is a functionalized nucleoside selected from the group consisting of:
a 2' functionalized nucleoside having cholic acid linked to the 2'-position of the nucleoside;
a heterocyclic base functionalized nucleoside having cholic acid linked to the heterocyclic base of the nucleoside;
a 5' terminal nucleoside having cholic acid linked to the 5'-position of the nucleoside;
a 3' terminal nucleoside having cholic acid linked to the 3'-position of the nucleoside; and
an inter-strand nucleoside having cholic acid linked to an inter-nucleotide linkage linking said inter-strand nucleoside to an adjacent nucleoside.

6. A compound comprising a plurality of linked nucleosides, wherein:
at least one of the nucleosides is a 2'-deoxy'-2'-fluoro, 2'-O-C₁-C₂₀-alkyl, 2'-O-C₂-C₂₀-alkenyl, 2'-O-C-₂-C₂₀-alkynyl, 2'-S-C₁-C-₂₀-alkyl, 2'-S-C₂-C₂₀-alkenyl, 2'-S-C₂-C₂₀-alkynyl, 2'-NH-C₁-C₂₀-alkyl, 2'NH-C₂-C₂₀-alkenyl, 2'-NH-C₂-C₂₀-alkynyl nucleoside; and
at least one of the nucleosides is a functionalized nucleoside selected from the group comprising:
(i) a 2'-functionalized nucleoside having a steroid molecule, a reporter enzyme, a peptide, a protein, a water soluble vitamin, a lipid soluble vitamin, an RNA cleaving complex, a metal chelator, a porphyrin, an alkylator, a hybrid photonuclease/intercalator, or an aryl azide photo-crosslinking agent, directly linked to the 2'-position of the nucleoside;
(ii) a 2'-functionalized nucleoside having a reporter enzyme, a peptide, a protein, a water soluble vitamin, a lipid soluble vitamin, an RNA cleaving complex, a metal chelator, an alkylator, or a hybrid photonuclease/intercalator linked, through a linking moiety, to the 2'-position of the nucleoside, provided that said linking moiety does not include a disulfide group;
(iii) a heterocyclic base functionalized nucleoside having a steroid molecule, a reporter molecule, a reporter enzyme, a peptide, a protein, a water soluble vitamin, a lipid soluble vitamin, a metal chelator, a porphyrin, an alkylator, a hybrid photonuclease/intercalator, or an aryl azide photo-crosslinking agent linked to the heterocyclic base of the nucleoside;
(iv) a 5' terminal nucleoside having a steroid molecule, a reporter molecule, a reporter enzyme, a peptide, a protein, a water soluble vitamin, a lipid soluble vitamin, a metal chelator, a porphyrin, an alkylator, a hybrid photonuclease/intercalator, or an aryl azide photo-crosslinking agent directly linked to the 5'-position of the nucleoside;
(v) a 5' terminal nucleoside having a steroid molecule, a reporter molecule, a non-aromatic lipophilic molecule, a reporter enzyme, a peptide, a protein, a water soluble vitamin, a lipid soluble vitamin, an RNA cleaving complex, a metal chelator, a porphyrin, an alkylator, a hybrid photonuclease/intercalator, or an aryl azide photo-crosslinking agent linked, through a linking moiety, to the 5'-position of the nucleoside, provided that said linking moiety does not include a disulfide group;
(vi) a 3' terminal nucleoside having a steroid molecule, a reporter molecule, a non-aromatic lipophilic molecule, a reporter enzyme, a peptide, a protein, a water soluble vitamin, a lipid soluble vitamin, an RNA cleaving complex, a metal chelator, a porphyrin, an alkylator, a hybrid photonuclease/intercalator, or an aryl azide photo-crosslinking agent directly linked to the 3'-position of the nucleoside;
(vii) a 3' terminal nucleoside having a steroid molecule, a reporter molecule, a non-aromatic lipophilic molecule, a reporter enzyme, a peptide, a protein, a water soluble vitamin, a lipid soluble vitamin, an RNA cleaving complex, a metal chelator, a porphyrin, an alkylator, a hybrid photonuclease/intercalator, or an aryl azide photo-crosslinking agent linked, through a linking moiety, to the 3'-position of the nucleoside, provided that said linking moiety does not include a disulfide group;
(viii) an inter-strand nucleoside having a steroid molecule, a reporter molecule, a non-aromatic lipophilic molecule, a reporter enzyme, a peptide, a protein, a water soluble vitamin, a lipid soluble vitamin, an RNA cleaving complex, a metal chelator, a porphyrin, an alkylator, a hybrid photonuclease/intercalator, or an aryl azide photo-crosslinking agent linked to an inter-strand linkage linking said inter-strand nucleoside to an adjacent nucleoside.

7. The compound of any one of the preceding claims, wherein at least two of the linked nucleosides are linked by phosphorothioate linking groups.

8. A compound for use in medicine having a plurality of linked nucleosides, wherein said compound includes at least one nucleoside functionalized at its 2'-position by attachment, through a linking moiety, of a reporter molecule, a reporter enzyme, a peptide, a protein, a water soluble vitamin, or a lipid soluble vitamin, provided that said linking moiety does not include a disulfide group.

9. A method comprising:
functionalizing at least one nucleoside of an oligonucleotide at its 2'-position by direct attachment of a steroid molecule, a reporter enzyme, a peptide, a protein, a water soluble vitamin, a lipid soluble vitamin, a metal chelator, a porphyrin, an alkylator, a hybrid photonuclease/intercalator, or an aryl azide photo-crosslinking agent; and
further functionalizing at least one nucleoside of said oligonucleotide at its 2'-position by attachment through a linking moiety of a reporter enzyme, a peptide, a protein, a water soluble vitamin, or a lipid soluble vitamin, provided that said linking moiety does not include a disulfide group.

10. A compound comprising a plurality of linked nucleosides, wherein at least one of the nucleosides is a functionalized nucleoside selected from the group consisting of:
a 2' functionalized nucleoside having folic acid linked to the 2'-position of the nucleoside;
a heterocyclic base functionalized nucleoside having folic acid linked to the heterocyclic base of the nucleoside;
a 5' terminal nucleoside having folic acid linked to the 5'-position of the nucleoside;
a 3' terminal nucleoside having folic acid linked to the 3'-position of the nucleoside; and
an inter-strand nucleoside having folic acid linked to an inter-nucleotide linkage linking said inter-strand nucleoside to an adjacent nucleoside.

11. A compound comprising a plurality of linked nucleosides, wherein at least one of the nucleosides is a functionalized nucleoside selected from the group consisting of:
a 2' functionalized nucleoside having pyrene directly linked to the 2'-position of the nucleoside;
a 5' terminal nucleoside having pyrene linked to the 5'-position of the nucleoside;
a 3' terminal nucleoside having pyrene linked to the 3'-position of the nucleoside; and
an inter-strand nucleoside having pyrene linked to an inter-nucleotide linkage linking said inter-strand nucleoside to an adjacent nucleoside.

12. A compound according to any one of the claims 1 to 7 and claim 10 for use in medicine.

13. Use of a compound according to any one of claims 1 to 7 and claim 10 for the manufacture of a medicament for assisting in the transfer of the antisense therapeutic or diagnostic agent across the cellular membrane.

## Patentansprüche

1. Verbindung, die eine Vielzahl von verknüpften Nukleosiden umfaßt, wobei
mindestens eines der Nukleoside an seiner 2'-Stellung durch direkte Bindung eines Substituenten, ausgewählt aus der Gruppe, bestehend aus einem Steroidmolekül, einem Reporterenzym, einem Peptid, einem Protein, einem Pyren, einem wasserlöslichen Vitamin, einem fettlöslichen Vitamin, einem Metallkomplexbildner, einem Porphyrin, einem Alkylierungsmittel, einem Hybrid aus Photonuklease/Interkalierungsmittel und einem Arylazid-Photovernetzungsmittel, funktionalisiert ist oder
mindestens eines der Nukleoside an seiner 2'-Stellung durch Bindung eines Substituenten, ausgewählt aus der Gruppe, bestehend aus einem Reporterenzym, einem Peptid, einem Protein, einem wasserlöslichen Vitamin, einem fettlöslichen Vitamin, einem RNA spaltenden Komplex, einem Metallkomplexbildner, einem Alkylierungsmittel und einem Hybrid aus Photonuklease/Interkalierungsmittel, über eine verknüpfenden Einheit funktionalisiert ist, mit der Maßgabe, daß die verknüpfende Einheit keine Disulfidgruppe einschließt.

2. Verbindung nach Anspruch 1, wobei das funktionalisierte Nukleosid ein Steroidmolekül, Biotin, ein Reporterenzym, ein Peptid, ein Protein, ein Pyren, Thiamin, Riboflavin, Nikotinsäure, Niacin, Pyridoxal, Panthothensäure, Biotin, Folsäure, Methylfolat, ein Vitamin B₁₂ Cobamin-Coenzym, Inositol, Cholin, Ascorbinsäure, Retinolsäure, Retinol, ein Tocopherol, Vitamin D, Vitamin K, EDTA, DTPA, o-Phenanthrolin, ein Photonuklease/Interkalierungsmittel, ein Arylazid-Photovernetzungsmittel, einen Phenanthrolin/Metallkomplex oder einen Bipyridin/Metallkomplex, gebunden an die 2'-Stellung des Nukleosids, umfaßt.

3. Verbindung nach Anspruch 1 oder Anspruch 2, wobei die verknüpfende Einheit eine Ω-Aminoalkoxy- oder Ω-Aminoalkylaminoeinheit umfaßt.

4. Verbindung nach Anspruch 1, wobei Cholsäure, Deoxycholsäure, Dehydrocholsäure, Cortison, Testosteron, Cholesterin oder Digoxigenin an die 2'-Stellung des Nukleosids gebunden ist.

5. Verbindung, die eine Vielzahl von verknüpften Nukleosiden umfaßt, wobei mindestens eines der Nukleoside ein funktionalisiertes Nukleosid ist, ausgewählt aus der Gruppe, bestehend aus
einem 2'-funktionalisierten Nukleosid mit Cholsäure, gebunden an die 2'-Stellung des Nukleosids,
einem mit einer heterocyclischen Base funktionalisierten Nukleosid mit Cholsäure, gebunden an die heterocyclische Base des Nukleosids,
einem 5'-terminalen Nukleosid mit Cholsäure, gebunden an die 5'-Stellung des Nukleosids,
einem 3'-terminalen Nukleosid mit Cholsäure, gebunden an die 3'-Stellung des Nukleosids, und
einem Zwischenstrangnukleosid mit Cholsäure, gebunden an eine Zwischennukleotidverknüpfung, welche das Zwischenstrangnukleosid an ein benachbartes Nukleosid bindet.

6. Verbindung, die eine Vielzahl von verknüpften Nukleosiden umfaßt, wobei mindestens eines der Nukleoside ein 2'-Deoxy'-2'-fluor-, 2'-O-C₁-C₂₀-Alkyl-, 2'-O-C₂-C₂₀-Alkenyl-, 2'-O-C-₂-C₂₀-Alkinyl-, 2'-S-C₁-C-₂₀-Alkyl-, 2'-S-C₂-C₂₀-Alkenyl-, 2'-S-C₂-C₂₀-Alkinyl-, 2'-NH-C₁-C₂₀-Alkyl-, 2'NH-C₂-C₂₀-Alkenyl-, 2'-NH-C₂-C₂₀-Alkinylnukleosid ist und
mindestens eines der Nukleoside ein funktionalisiertes Nukleosid ist, ausgewählt aus der Gruppe, umfassend
(i) ein 2'-funktionalisiertes Nukleosid mit einem Steroidmolekül, einem Reporterenzym, einem Peptid, einem Protein, einem wasserlöslichen Vitamin, einem fettlöslichen Vitamin, einem RNA spaltenden Komplex, einem Metallkomplexbildner, einem Porphyrin, einem Alkylierungsmittel, einem Hybrid aus Photonuklease/Interkalierungsmittel oder einem Arylazid-Photovernetzungsmittel, gebunden direkt an die 2'-Stellung des Nukleosids,
(ii) ein 2'-funktionalisiertes Nukleosid mit einem Reporterenzym, einem Peptid, einem Protein, einem wasserlöslichen Vitamin, einem fettlöslichen Vitamin, einem RNA spaltenden Komplex, einem Metallkomplexbildner, einem Alkylierungsmittel oder einem Hybrid aus Photonuklease/lnterkalierungsmittel, gebunden über eine verknüpfende Einheit an die 2'-Stellung des Nukleosids, mit der Maßgabe, daß die verknüpfende Einheit keine Disulfidgruppe einschließt,
(iii) ein mit einer heterocyclischen Base funktionalisiertes Nukleosid mit einem Steroidmolekül, einem Reportermolekül, einem Reporterenzym, einem Peptid, einem Protein, einem wasserlöslichen Vitamin, einem fettlöslichen Vitamin, einem Metallkomplexbildner, einem Porphyrin, einem Alkylierungsmittel, einem Hybrid aus Photonuklease/Interkalierungsmittel oder einem Arylazid-Photovernetzungsmittel, gebunden an die heterocyclische Base des Nukleosids,
(iv) ein 5'-terminales Nukleosid mit einem Steroidmolekül, einem Reportermolekül, einem Reporterenzym, einem Peptid, einem Protein, einem wasserlöslichen Vitamin, einem fettlöslichen Vitamin, einem Metallkomplexbildner, einem Porphyrin, einem Alkylierungsmittel, einem Hybrid aus Photonuklease/Interkalierungsmittel oder einem Arylazid-Photovernetzungsmittel, gebunden direkt an die 5'-Stellung des Nukleosids,
(v) ein 5'-terminales Nukleosid mit einem Steroidmolekül, einem Reportermolekül, einem nichtaromatischen, lipophilen Molekül, einem Reporterenzym, einem Peptid, einem Protein, einem wasserlöslichen Vitamin, einem fettlöslichen Vitamin, einem RNA spaltenden Komplex, einem Metallkomplexbildner, einem Porphyrin, einem Alkylierungsmittel, einem Hybrid aus Photonuklease/Interkalierungsmittel oder einem Arylazid-Photovernetzungsmittel, gebunden über eine verknüpfende Einheit an die 5'-Stellung des Nukleosids, mit der Maßgabe, daß die verknüpfende Einheit keine Disulfidgruppe einschließt,
(vi) ein 3'-terminales Nukleosid mit einem Steroidmolekül, einem Reportermolekül, einem nichtaromatischen, lipophilen Molekül, einem Reporterenzym, einem Peptid, einem Protein, einem wasserlöslichen Vitamin, einem fettlöslichen Vitamin, einem RNA spaltenden Komplex, einem Metallkomplexbildner, einem Porphyrin, einem Alkylierungsmittel, einem Hybrid aus Photonuklease/Interkalierungsmittel oder einem Arylazid-Photovemetzungsmittel, gebunden direkt an die 3'-Stellung des Nukleosids,
(vii) ein 3'-terminales Nukleosid mit einem Steroidmolekül, einem Reportermolekül, einem nichtaromatischen, lipophilen Molekül, einem Reporterenzym, einem Peptid, einem Protein, einem wasserlöslichen Vitamin, einem fettlöslichen Vitamin, einem RNA spaltenden Komplex, einem Metallkomplexbildner, einem Porphyrin, einem Alkylierungsmittel, einem Hybrid aus Photonuklease/Interkalierungsmittel oder einem Arylazid-Photovernetzungsmittel, gebunden über eine verknüpfende Einheit an die 3'-Stellung des Nukleosids, mit der Maßgabe, daß die verknüpfende Einheit keine Disulfidgruppe einschließt,
(viii) ein Zwischenstrangnukleosid mit einem Steroidmolekül, einem Reportermolekül, einem nichtaromatischen, lipophilen Molekül, einem Reporterenzym, einem Peptid, einem Protein, einem wasserlöslichen Vitamin, einem fettlöslichen Vitamin, einem RNA spaltenden Komplex, einem Metallkomplexmittel, einem Porphyrin, einem Alkylierungsmittel, einem Hybrid aus Photonuklease/Interkalierungsmittel oder einem Arylazid-Photovernetzungsmittel, gebunden an eine Zwischenstrangverknüpfung, welche das Zwischenstrangnukleosid an ein benachbartes Nukleosid bindet.

7. Verbindung nach einem der vorhergehenden Ansprüche, wobei mindestens zwei der gebundenen Nukleoside durch Phosphorthioatverknüpfungsgruppen gebunden sind.

8. Verbindung zur Verwendung in der Medizin mit einer Vielzahl von verknüpften Nukleosiden, wobei die Verbindung mindestens ein Nukleosid einschließt, das an seiner 2'-Stellung durch Bindung eines Reportermoleküls, eines Reporterenzyms, eines Peptids, eines Proteins, eines wasserlöslichen Vitamins oder eines fettlöslichen Vitamins, über eine verknüpfende Einheit funktionalisiert ist, mit der Maßgabe, daß die verknüpfende Einheit keine Disulfidgruppe einschließt.

9. Verfahren, umfassend
das Funktionalisieren von mindestens einem Nukleosid eines Oligonukleotids an seiner 2'-Position durch direkte Bindung eines Steroidmoleküls, eines Reporterenzyms, eines Peptids, eines Proteins, eines wasserlöslichen Vitamins, eines fettlöslichen Vitamins, eines Metallkomplexbildners, eines Porphyrins, eines Alkylierungsmittels, eines Hybrids aus Photonuklease/Interkalierungsmittel oder eines Arylazid-Photovernetzungsmittels und
weiter das Funktionalisieren von mindestens einem Nukleosid des Oligonukleotids an seiner 2'-Stellung durch Bindung eines Reporterenzyms, eines Peptids, eines Proteins, eines wasserlöslichen Vitamins oder eines fettlöslichen Vitamins über eine verknüpfende Einheit, mit der Maßgabe, daß die verknüpfende Einheit keine Disulfidgruppe einschließt.

10. Verbindung, die eine Vielzahl von verknüpften Nukleosiden umfaßt, wobei mindestens eines der Nukleoside ein funktionalisiertes Nukleosid ist, ausgewählt aus der Gruppe, bestehend aus
einem 2'-funktionalisierten Nukleosid mit Folsäure, gebunden an die 2'-Stellung des Nukleosids,
einem mit einer heterocyclischen Base funktionalisierten Nukleosid mit Folsäure, gebunden an die heterocyclische Base des Nukleosids,
einem 5'-terminalen Nukleosid mit Folsäure, gebunden an die 5'-Stellung des Nukleosids,
einem 3'-terminalen Nukleosid mit Folsäure, gebunden an die 3'-Stellung des Nukleosids, und
einem Zwischenstrangnukleosid mit Folsäure, gebunden an eine Zwischennukleotidverknüpfung, welche das Zwischenstrangnukleosid an ein benachbartes Nukleosid bindet.

11. Verbindung, die eine Vielzahl von verknüpften Nukleosiden umfaßt, wobei mindestens eines der Nukleoside ein funktionalisiertes Nukleosid ist, ausgewählt aus der Gruppe, bestehend aus
einem 2'-funktionalisierten Nukleosid mit Pyren, gebunden direkt an die 2'-Stellung des Nukleosids,
einem 5'-terminalen Nukleosid mit Pyren, gebunden an die 5'-Stellung des Nukleosids,
einem 3'-terminalen Nukleosid mit Pyren, gebunden an die 3'-Position des Nukleosids, und
einem Zwischenstrangnukleosid mit Pyren, gebunden an eine Zwischennukleotidverknüpfung, welche das Zwischenstrangnukleosid an ein benachbartes Nukleosid bindet.

12. Verbindung nach einem der Ansprüche 1 bis 7 und Anspruch 10 zur Verwendung in der Medizin.

13. Verwendung einer Verbindung nach einem der Ansprüche 1 bis 7 und Anspruch 10 zur Herstellung eines Medikaments zur Unterstützung im Transfer des therapeutischen oder diagnostischen Antisense-Mittels durch die zelluläre Membran.

## Revendications

1. Composé comprenant une pluralité de nucléosides liés, dans lequel :
au moins un desdits nucléosides est fonctionnalisé à sa position 2' par fixation directe d'un substituant choisi dans le groupe constitué par une molécule stéroïde, une enzyme reporteur, un peptide, une protéine, un pyrène, une vitamine hydrosoluble, une vitamine liposoluble, un chélateur de métaux, une porphyrine, un alkylateur, un hybride photonucléase/intercalateur, et un agent photoréticulant arylazide, ou
au moins un des dits nucléosides est fonctionnalisé à sa position 2' par fixation, via un groupement de liaison, d'un substituant choisi dans le groupe constitué par une enzyme reporteur, un peptide, une protéine, une vitamine hydrosoluble, une vitamine liposoluble, un complexe de clivage d'ARN, un chélateur de métaux, un alkylateur, et un hybride photonucléase/intercalateur, sous réserve que ledit groupement de liaison ne comprenne pas un groupe disulfure.

2. Composé selon la revendication 1, dans lequel ledit nucléoside fonctionnalisé comprend une molécule stéroïde, une biotine, une enzyme reporteur, un peptide, une protéine, un pyrène, la thiamine, la riboflavine, l'acide nicotinique, la niacine, le pyridoxal, l'acide pantothénique, la biotine, l'acide folique, le folate de méthyle, une coenzyme cobamide de vitamine B₁₂, l'inositol, la choline, l'acide ascorbique, l'acide rétinoïque, le rétinol, le tocophérol, la vitamine D, la vitamine K, l'EDTA, la DTPA, la o-phénanthroline, un photonucléase/intercalateur, un arylazide photoréticulant, un complexe phénanthroline/métal ou un complexe bipyridine/métal lié en position 2' dudit nucléoside.

3. Composé selon la revendication 1 ou la revendication 2, dans lequel ledit groupement de liaison comprend un groupement Ω-aminoalcoxy ou Ω-aminoalkylamino.

4. Composé selon la revendication 1, dans lequel l'acide cholique, l'acide déoxycholique, l'acide déhydrocholique, la cortisone, la testostérone, le cholestérol ou la digoxigénine est lié en position 2' dudit nucléoside.

5. Composé comprenant une pluralité de nucléosides liés, dans lequel au moins un des nucléosides est un nucléoside fonctionnalisé choisi dans le groupe constitué par :
un nucléoside fonctionnalisé en 2' ayant l'acide cholique lié en position 2' du nucléoside ;
un nucléoside fonctionnalisé par une base hétérocyclique ayant l'acide cholique lié à la base hétérocyclique du nucléoside ;
un nucléoside à terminaison 5' ayant l'acide cholique lié en position 5' du nucléoside ;
un nucléoside à terminaison 3' ayant l'acide cholique lié en position 3' du nucléoside ; et
un nucléoside inter-brin ayant l'acide cholique lié à une liaison inter-nucléotide liant ledit nucléoside inter-brin à un nucléoside adjacent.

6. Composé comprenant une pluralité de nucléosides liés, dans lequel :
au moins un des nucléosides est un 2'-déoxy-2'-fluoro, 2'-O-alkyl en C₁-C₂₀, 2'-O-alcényl en C₂-C₂₀, 2'-O-alcynyl en C₂-C₂₀, 2'-S-alkyl en C₁-C₂₀, 2'-S-alcényl en C₂-C₂₀, 2'-S-alcynyl en C₂-C₂₀, 2'-NH-alkyl en C₁-C₂₀, 2'-NH-alcényl en C₂-C₂₀, 2'-NH-alcynyl en C₂-C₂₀ nucléoside ; et
au moins un des nucléosides est un nucléoside fonctionnalisé choisi dans le groupe comprenant :
(i) un nucléoside fonctionnalisé en 2' ayant une molécule de stéroïde, une enzyme reporteur, un peptide, une protéine, une vitamine hydrosoluble, une vitamine liposoluble, un complexe de clivage d'ARN, un chélateur de métaux, une porphyrine, un alkylateur, un hybride photonucléase/intercalateur, ou un agent photoréticulant arylazide, directement lié en position 2' du nucléoside ;
(ii) un nucléoside fonctionnalisé en 2' ayant une enzyme reporteur, un peptide, une protéine, une vitamine hydrosoluble, une vitamine liposoluble, un complexe de clivage d'ARN, un chélateur de métaux, un alkylateur, ou un hybride photonucléase/intercalateur lié, via un groupement de liaison, en position 2' du nucléoside, sous réserve que ledit groupement de liaison ne comprenne pas un groupe disulfure ;
(iii) un nucléoside fonctionnalisé par une base hétérocyclique ayant une molécule de stéroïde, une molécule reporteur, une enzyme reporteur, un peptide, une protéine, une vitamine hydrosoluble, une vitamine liposoluble, un chélateur de métaux, une porphyrine, un alkylateur, un hybride photonucléase/intercalateur, ou un agent photoréticulant arylazide, lié à la base hétérocyclique du nucléoside;
(iv) un nucléoside à terminaison 5' ayant une molécule de stéroïde, une molécule reporteur, une enzyme reporteur, un peptide, une protéine, une vitamine hydrosoluble, une vitamine liposoluble, un chélateur de métaux, une porphyrine, un alkylateur, un hybride photonucléase/intercalateur, ou un agent photoréticulant arylazide, directement lié en position 5' du nucléoside ;
(v) un nucléoside à terminaison 5' ayant une molécule de stéroïde, une molécule reporteur, une molécule lipophile non aromatique, une enzyme reporteur, un peptide, une protéine, une vitamine hydrosoluble, une vitamine liposoluble, un complexe de clivage d'ARN, un chélateur de métaux, une porphyrine, un alkylateur, un hybride photonucléase/intercalateur, ou un agent photoréticulant arylazide lié, via un groupement de liaison, en position 5' du nucléoside, sous réserve que ledit groupement de liaison ne comprenne pas un groupe disulfure ;
(vi) un nucléoside à terminaison 3' ayant une molécule de stéroïde, une molécule reporteur, une molécule lipophile non aromatique, une enzyme reporteur, un peptide, une protéine, une vitamine hydrosoluble, une vitamine liposoluble, un complexe de clivage d'ARN, un chélateur de métaux, une porphyrine, un alkylateur, un hybride photonucléase/intercalateur, ou un agent photoréticulant arylazide, directement lié en position 3' du nucléoside ;
(vii) un nucléoside à terminaison 3' ayant une molécule de stéroïde, une molécule reporteur, une molécule lipophile non aromatique, une enzyme reporteur, un peptide, une protéine, une vitamine hydrosoluble, une vitamine liposoluble, un complexe de clivage d'ARN, un chélateur de métaux, une porphyrine, un alkylateur, un hybride photonucléase/intercalateur, ou un agent photoréticulant arylazide lié, via un groupement de liaison, en position 3' du nucléoside, sous réserve que ledit groupement de liaison ne comprenne pas un groupe disulfure ;
(viii) un nucléoside inter-brin ayant une molécule de stéroïde, une molécule reporteur, une molécule lipophile non aromatique, une enzyme reporteur, un peptide, une protéine, une vitamine hydrosoluble, une vitamine liposoluble, un complexe de clivage d'ARN, un chélateur de métaux, une porphyrine, un alkylateur, un hybride photonucléase/intercalateur, ou un agent photoréticulant arylazide lié à une liaison inter-brin liant ledit nucléoside inter-brin à un nucléoside adjacent.

7. Composé selon l'une quelconque des revendications précédentes, dans lequel au moins deux des nucléosides liés sont liés par des groupes de liaison phosphorothioate.

8. Composé pour l'utilisation en médecine ayant une pluralité de nucléosides liés, dans lequel le composé comprend au moins un nucléoside fonctionnalisé à sa position 2' par fixation, via un groupement de liaison, d'une molécule reporteur, d'une enzyme reporteur, d'un peptide, d'une protéine, d'une vitamine hydrosoluble ou d'une vitamine liposoluble, sous réserve que ledit groupement de liaison ne comprenne pas un groupe disulfure.

9. Procédé comprenant les étapes suivantes consistant à:
fonctionnaliser au moins un nucléoside d'un oligonucléotide à sa position 2' par fixation directe d'une molécule stéroïde, d'une enzyme reporteur, d'un peptide, d'une protéine, d'une vitamine hydrosoluble, d'une vitamine liposoluble, d'un chélateur de métaux, d'une porphyrine, d'un alkylateur, d'un hybride photonucléase/intercalateur, ou d'un agent photoréticulant arylazide ; et
en outre fonctionnaliser au moins un nucléoside dudit oligonucléotide à sa position 2' par fixation via un groupement de liaison, d'une enzyme reporteur, d'un peptide, d'une protéine, d'une vitamine hydrosoluble ou d'une vitamine liposoluble, sous réserve que ledit groupement de liaison ne comprenne pas un groupe disulfure.

10. Composé comprenant une pluralité de nucléosides liés, dans lequel au moins un des nucléosides est un nucléoside fonctionnalisé choisi dans le groupe constitué par :
un nucléoside fonctionnalisé en 2' ayant l'acide folique lié en position 2' du nucléoside ;
un nucléoside fonctionnalisé par une base hétérocyclique ayant l'acide folique lié à la base hétérocyclique du nucléoside ;
un nucléoside à terminaison 5' ayant l'acide folique lié en position 5' du nucléoside ;
un nucléoside à terminaison 3' ayant l'acide folique lié en position 3' du nucléoside ; et
un nucléoside inter-brin ayant l'acide folique lié à une liaison inter-nucléotide liant ledit nucléoside inter-brin à un nucléoside adjacent.

11. Composé comprenant une pluralité de nucléosides liés, dans lequel au moins un des nucléosides est un nucléoside fonctionnalisé choisi dans le groupe constitué par :
un nucléoside fonctionnalisé en 2' ayant le pyrène directement lié en position 2' du nucléoside ;
un nucléoside à terminaison 5' ayant le pyrène lié en position 5' du nucléoside ;
un nucléoside à terminaison 3' ayant le pyrène lié en position 3' du nucléoside ; et
un nucléoside inter-brin ayant le pyrène lié à une liaison inter-nucléotide liant ledit nucléoside inter-brin à un nucléoside adjacent.

12. Composé selon l'une quelconque des revendications 1 à 7 et la revendication 10 pour l'utilisation en médecine.

13. Utilisation d'un composé selon l'une quelconque des revendications 1 à 7 et la revendication 10 pour la fabrication d'un médicament pour l'assistance dans le transfert de l'agent d'antisens thérapeutique ou diagnostique à travers la membrane cellulaire.
